# FASCICULE DE BREVET EUROPEEN

(11) **EP 2 049 077 B1**
(45) Date de publication et mention de la délivrance du brevet: **01.12.2010**
(21) Numéro de dépôt: 07823535.5
(22) Date de dépôt: 02.07.2007
(51) Int. Cl.: A61K 8/73, A61K 8/60, A61Q 19/00, A61Q 19/08

(54) **COMPOSITION COMPRENANT AU MOINS UN DERIVE C-GLYCOSIDE ET AU MOINS UN ACIDE HYALURONIQUE ET SON UTILISATION COSMETIQUE**
ZUSAMMENSETZUNG MIT MINDESTENS EINEM C-GLYCOSID-DERIVAT UND MINDESTENS EINER HYALURONSÄURE SOWIE IHRE KOSMETISCHE ANWENDUNG
COMPOSITION COMPRISING AT LEAST ONE C-GLYCOSIDE DERIVATIVE AND AT LEAST ONE HYALURONIC ACID AND ITS COSMETIC USE

(30) Priorité: 03.07.2006 US 817683 P; 12.03.2007 US 894254 P
(43) Date de publication de la demande: 22.04.2009
(73) Titulaire: L'Oréal, 75008 Paris (FR)
(72) Inventeur: BISSEY, Laure, F-75013 Paris (FR); LABOUREAU, Julien, F-75011 Paris (FR); SENEE, Jérome, F-91510 Lardy (FR); SAUSSAY, Sophie, F-95470 Survilliers (FR)
(74) Mandataire: Tanty, François
(86) Numéro de dépôt international: PCT/FR2007/051579
(87) Numéro de publication internationale: WO 2008/020140

(56) Documents cités:
- WO-A-02/051828

## Description

La présente invention se rapporte au domaine du soin de la peau et/ou de ses annexes.

L'invention a pour objet une composition cosmétique et/ou dermatologique topique comprenant, dans un milieu physiologiquement acceptable, au moins un acide hyaluronique ou l'un de ses dérivés et au moins un dérivé C-glycoside.

La présente invention porte aussi sur un procédé de traitement cosmétique de la peau et/ou de ses annexes, destiné à améliorer la fonction barrière comprenant au moins une étape consistant à appliquer sur la peau au moins une composition telle que définie ci-dessus.

En particulier, la composition de l'invention est destinée à améliorer la fonction barrière de la peau. Elle trouve ainsi une utilisation dans l'hydratation de la peau, dans l'amélioration de la souplesse de la peau, dans l'amélioration et/ou la diminution du microrelief de la peau, ainsi que dans la lutte contre les signes cutanés du vieillissement.

La peau humaine est constituée de deux compartiments à savoir un compartiment profond, le derme et un compartiment superficiel, l'épiderme.

Le derme fournit à l'épiderme un support solide. C'est également son élément nourricier. Il est principalement constitué de fibroblastes et d'une matrice extracellulaire composée elle-même principalement de collagène, d'élastine et d'une substance, dite substance fondamentale, composants synthétisés par le fibroblaste. On y trouve aussi des leucocytes, des mastocytes ou encore des macrophages tissulaires. Il contient également des vaisseaux sanguins et des fibres nerveuses.

L'épiderme est en contact avec l'environnement extérieur.

L'épiderme humain naturel est composé principalement de trois types de cellules qui sont les kératinocytes, très majoritaires, les mélanocytes et les cellules de Langerhans.

Les cellules constituant l'épiderme sont délimitées par un domaine lipidique intercellulaire.

Chacun de ces types cellulaires contribue par ses fonctions propres au rôle essentiel joué dans l'organisme par la peau. En particulier, les kératinocytes subissent un processus de maturation continu et orienté qui, des kératinocytes se trouvant dans la couche basale de l'épiderme, aboutit à la formation de cornéocytes, qui sont des cellules mortes totalement kératinisées constituées de kératinocytes au stade terminal de leur différenciation.

Au cours de la différenciation, les phospholipides dont le rôle consiste à élaborer la structure fluide des membranes cellulaires des couches vivantes de l'épiderme, sont peu à peu remplacés par un mélange composé en majeure partie d'acides gras, de cholestérol et de sphingolipides (céramides). Ces lipides, qui sont organisés en phases cristal liquide lamellaires spécifiques, forment le ciment intracellulaire du *stratum corneum* et sont essentiels pour les échanges en eau et la fonction barrière de l'épiderme. Ainsi, la structure lamellaire des lipides du domaine lipidique de l'épiderme et les cornéocytes participent à la fonction barrière épidermique.

La peau constitue ainsi une barrière contre les agressions extérieures, notamment chimiques, mécaniques ou infectieuses, et à ce titre un certain nombre de réactions de défense contre les facteurs environnementaux (climat, rayons ultraviolets, tabac, ...) et/ou les xénobiotiques, comme par exemple les micro-organismes, se produisent à son niveau.

Cette propriété, appelée fonction barrière, est principalement assurée par la couche la plus superficielle de l'épiderme, à savoir la couche cornée, appelée le *stratum corneum*.

Il est manifeste que la qualité et l'équilibre de la barrière cutanée et des muqueuses est dépendante de mécanismes biologiques endogènes complexes faisant intervenir de nombreux facteurs de croissance, des molécules d'adhésion, des hormones et des enzymes du métabolisme lipidique.

Ainsi une altération de la barrière cutanée peut se produire en présence d'agressions externes de type agents irritants (détergents, acides, bases, oxydants, réducteurs, solvants concentrés, gaz ou fumées toxiques), sollicitations mécaniques (frottements, chocs, abrasion, arrachement de la surface, projection de poussières, de particules, rasage ou épilation), déséquilibres thermiques ou climatiques (froid, sécheresse, radiations), xénobiotiques (micro-organismes indésirable, allergènes) ou d'agressions internes de type stress psychologique.

Des personnes plus particulièrement concernées par cette altération de la fonction barrière par des agressions extérieures peuvent être les suivantes :
- les personnes à peau dite « fragile » ou « délicate » et vulnérable se déséquilibrant rapidement lors de variations de la température ou de l'humidité relative de grande amplitude (cas des peaux de bébé par exemple) ;
- les personnes à peau dite « fragilisée », regroupant notamment
   - les personnes dont le film hydro-lipidique protecteur composé de sueur, de sébum et de facteurs d'hydratation naturelle se raréfie, comme c'est le cas pour les personnes âgées de plus de 60 ans et notamment dans le cadre du grand âge (au moins 75 ans) ;
   - les personnes dont la composition du film hydro-lipidique est modifiée, comme c'est le cas des personnes diabétiques, ou dialysées, ou atteintes de certaines maladies ;
- les personnes qui possèdent un seuil de réactivité abaissé du à une hyperactivité neurogène ; ces peaux vont donc présenter ces sensations et ces signes cliniques beaucoup plus rapidement et fréquemment que les autres types de peaux : ce sont les personnes à peaux sensibles.

On pourra également parler de personnes à peau « agressée » pour les peaux rasées par exemple.

Une altération de la fonction barrière cutanée peut notamment se traduire par un trouble de l'hydratation, par une perte de souplesse de la peau, par une altération de l'éclat du teint, par l'apparition d'une rugosité sur la peau et plus généralement par l'apparition de signes cutanés du vieillissement.

Il convient alors de chercher à augmenter la différenciation épidermique pour renforcer la fonction barrière de la peau.

En particulier, on cherche ainsi à améliorer et/ou renforcer la fonction barrière cutanée pour :
- pallier les problèmes d'hydratation de la peau et/ou de ses annexes, notamment des muqueuses, et notamment traiter les peaux sèches,
- améliorer la souplesse de la peau,
- maintenir et/ou améliorer l'éclat du teint,
- prévenir et/ou traiter la rugosité ou micro-relief de la peau pouvant se manifester par des marques de varicelle ou d'acné,
- estomper les signes cutanés et/ou les imperfections cutanées liées au vieillissement cutané parmi lesquels on peut citer l'altération des propriétés viscoélastiques ou biomécaniques de la peau, l'altération de la cohésion des tissus, l'amincissement de la peau, l'apparition de rides et/ou de ridules, l'apparition d'un brunissement et/ou d'un jaunissement de la peau, et l'apparition de tâches de vieillesse ou sénescence ou lentigo.

Les signes cutanés du vieillissement peuvent être d'origine actinique.

Les sucres et dérivés du sucre sont des produits déjà mis à profit à des fins diverses pour la formulation de compositions cosmétiques destinées tant au soin de la peau qu'au soin et/ou au lavage des fibres kératiniques.

Ainsi, dans le document WO 99/24009, le D-xylose et ses dérivés sont proposés à des fins de préparation de produits cosmétiques ou pharmaceutiques visant à améliorer la fonctionnalité des cellules de l'épiderme.

Parmi les sucres utilisables dans le domaine, les dérivés C-glycosides s'avèrent tout particulièrement intéressants. Certains dérivés C-glycosides ont notamment démontré des propriétés biologiques intéressantes, en particulier pour lutter contre le vieillissement de l'épiderme et/ou contre le dessèchement de la peau. De tels composés sont notamment décrits dans le document WO 02/051828.

Ces composés agissent par l'intermédiaire de la stimulation de la synthèse des glycosaminoglycannes contenant un résidu D-glucosamine et/ou N-acétyl-D-glucosamine et sont représentés par la formule : 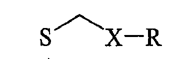 dans laquelle S représente un monosaccharide ou un polysaccharide, R représente différents radicaux linéaires ou cycliques et le groupement X peut représenter un groupement choisi parmi : -CO-, -CH(NR₁R₂)-, -CHR'-, -C(=CHR₃)- avec R₁, R₂, R' et R₃ pouvant représenter différent radicaux, dont le radical hydroxyle pour R₁, R₂ et R₃.

Toutefois, ces dérivés n'avaient jusqu'à présent jamais été utilisés à des fins d'amélioration et/ou de renforcement de la fonction barrière cutanée, ni encore moins en association avec l'acide hyaluronique.

L'acide hyaluronique est un glucosaminoglycanne majoritaire situé au niveau de la peau. Ainsi, les fibroblastes synthétisent majoritairement des collagènes, des glycoprotéines matricielles autres que les collagènes (fibronectine, laminine), des protéoglycannes et de l'élastine. Les kératinocytes synthétisent quant à eux majoritairement des glycosaminoglycannes sulfatés et de l'acide hyaluronique. L'acide hyaluronique est également appelé hyaluronanne (HA).

L'acide hyaluronique, est présent à l'état libre au niveau de l'épiderme et dans le derme et est responsable de la turgescence de la peau. Ce polysaccharide peut en effet retenir un grand volume d'eau, correspondant jusqu'à 1000 fois son poids. En ce sens l'acide hyaluronique joue un rôle important pour augmenter les quantités d'eau liées dans le tissu, ainsi que dans les propriétés mécaniques de la peau et dans la formation des rides.

Les inventeurs ont découvert que certains dérivés C-glycoside associés à l'acide hyaluronique ou l'un de ses dérivés permettaient d'améliorer et/ou de renforcer la fonction barrière de la peau.

Les inventeurs ont en particulier démontré que l'association conforme à l'invention présente un effet synergique sur l'activité de la transglutaminase kératinocytaire (TGk). Cette association permet ainsi une amélioration de l'homéostasie épidermique.

L'association conforme à l'invention présente la propriété d'activer la TGk, facteur essentiel de la différenciation terminale des kératinocytes et de sa cornéification.

La TGk est une enzyme appartenant à la famille des transpeptidases. C'est une enzyme calcium-dépendante qui catalyse la formation des ponts isopeptidiques ε(γ-glutamyl) lysine entre protéines épidermiques et favorise donc la formation des enveloppes cornées.

L'invention concerne ainsi selon un premier de ses aspects, une composition cosmétique et/ou dermatologique topique comprenant, dans un milieu physiologiquement acceptable, au moins un acide hyaluronique ou l'un de ses dérivés et au moins un dérivé C-glycoside.

Elle concerne également selon un autre de ses aspects, l'utilisation cosmétique d'une composition selon l'invention ou bien d'au moins un dérivé C-glycoside et d'au moins un acide hyaluronique ou l'un de ses dérivés dans une composition topique pour améliorer et/ou renforcer la fonction barrière cutanée de la peau.

Elle concerne également selon un autre de ses aspects l'utilisation cosmétique d'une composition selon l'invention ou bien d'au moins un dérivé C-glycoside et d'au moins un acide hyaluronique ou l'un de ses dérivés dans une composition topique, pour améliorer et/ou renforcer la protection de la peau vis-à-vis d'agressions extérieures.

Elle concerne en outre selon encore un autre de ses aspects, l'utilisation cosmétique d'une composition selon l'invention ou bien d'au moins un dérivé C-glycoside et d'au moins un acide hyaluronique ou l'un de ses dérivés dans des compositions topiques pour améliorer l'hydratation de la peau et/ou de ses annexes, prévenir et/ou traiter la rugosité ou le micro-relief et/ou améliorer l'éclat du teint et/ou pour améliorer la souplesse de la peau et/ou de ses annexes et/ou pour estomper les signes cutanés du vieillissement.

L'invention concerne également un procédé de traitement cosmétique destiné à améliorer et/ou renforcer la fonction barrière et/ou l'hydratation et/ou la résistance aux agressions extérieures de la peau et/ou de ses annexes et/ou pour lutter contre les signes cutanés du vieillissement et **caractérisé en ce qu**'on applique une quantité efficace d'au moins un dérivé C-glycoside d'au moins un acide hyaluronique ou l'un de ses dérivés ou bien une composition selon l'invention sur la peau et/ou ses annexes.

Plus particulièrement, le procédé vise à atténuer les signes cutanés du vieillissement d'origine actinique.

Il est notamment destiné aux personnes à peau mature, voire très mature.

Par « peaux matures » selon l'invention, on entend notamment des personnes ayant au moins 40 ans.

Par « peaux très matures » selon l'invention, on entend notamment personnes ayant au moins 50 ans, en particulier au moins 60 ans, voire 65 ans.

Selon un autre mode de réalisation, ladite composition peut être destinée à améliorer et/ou renforcer la fonction barrière d'une peau choisie parmi une peau fragile, une peau fragilisée, une peau agressée et/ou une peau sensible.

Dans le cadre de l'invention, la composition peut être utilisée pour l'application sur la peau saine, soumise ou pouvant être soumise à des agressions extérieures telles que rappelées ci-dessus. Dans d'autres cas particuliers, la composition de l'invention peut être appliquée sur la peau lorsqu'elle présente des signes cliniques de déficit de la barrière cutanée.

Ainsi l'invention a pour objet l'utilisation d'au moins un acide hyaluronique ou l'un de ses dérivés et d'au moins un dérivé C-glycoside, pour la préparation d'une composition topique notamment dermatologique destinée à améliorer et/ou renforcer la fonction barrière de la peau.

Un autre aspect de la présente invention est donc l'utilisation d'au moins un acide hyaluronique ou l'un de ses dérivés et d'au moins un dérivé C-glycoside, pour la préparation d'une composition topique notamment dermatologique destinée à améliorer et/ou renforcer la fonction barrière d'une peau lésée, en particulier d'une peau nécessitant une réparation et/ou une régénération tissulaire.

L'invention a enfin pour objet l'association d'au moins un acide hyaluronique ou d'au moins un de ses dérivés et d'au moins un dérivé C-glycoside pour son utilisation pour l'amélioration et/ou le renforcement de la fonction barrière d'une peau lésée, en particulier d'une peau nécessitant une réparation et/ou une régénération tissulaire.

La composition selon l'invention peut notamment être destinée à améliorer la cicatrisation ou stimuler la réparation et/ou la régénération tissulaire.

La réparation tissulaire peut s'avérer nécessaire dans tous les cas où la peau présente des lésions de toute origine, notamment dues aux dommages causés par les rayonnements UV ou les agressions extérieures de type pollution ou un stress.

Dans le cadre de la présente invention, on entend par «propriétés viscoélastiques ou biomécaniques de la peau » les propriétés d'extensibilité, de tonicité, de fermeté, de souplesse et/ou d'élasticité de la peau. Une peau manifestant une telle altération de ses propriétés viscoélastiques ou biomécaniques peut être qualifiée de peau molle, peau flasque, peau moins ferme, moins élastique ou encore peau affaissée.

L'altération de la « cohésion des tissus » se manifeste par l'altération des propriétés biomécaniques de la peau, l'amincissement de la peau et la diminution du métabolisme cellulaire de la peau.

Par « peau et/ou ses annexes », on entend notamment la peau, les muqueuses, les lèvres, le cuir chevelu, les cils, les sourcils et les cheveux.

Par « quantité efficace », on entend des quantités respectives de deux actifs qui permettent la manifestation de l'effet synergique recherché.

L'association selon l'invention pourra être formulée dans une même composition topique ou dans deux compositions topiques distinctes, pouvant être appliquées sur la peau et/ou ses annexes soit de façon simultanée, soit de façon successive ou décalée dans le temps.

### ACIDE HYALURONIQUE

Dans le cadre de la présente invention, le tenne « acide hyaluronique ou l'un de ses dérivés » recouvre notamment le motif de base de l'acide hyaluronique de formule :

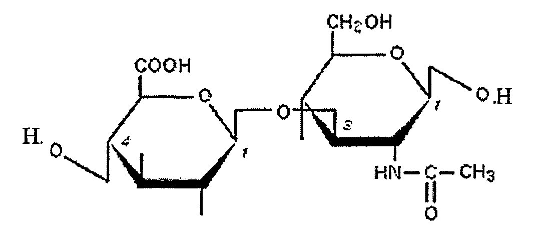

Il s'agit de la fraction la plus petite de l'acide hyaluronique comprenant un dimère disaccharidique, à savoir l'acide D-glucuronique et le N-acétyl glucosamine.

Le terme « acide hyaluronique ou l'un de ses dérivés » comprend aussi dans le cadre de la présente invention le polymère linéaire comprenant l'unité polymérique décrite ci-dessus, selon un enchaînement par les liaisons glycosidiques alternées β(1,4) et β(1,3), ayant un poids moléculaire (PM) pouvant varier entre 380 et 13 000 000 daltons. Ce poids moléculaire dépend en grande partie de la source d'obtention de l'acide hyaluronique et/ou des méthodes de préparation.

Le terme « acide hyaluronique ou l'un de ses dérivés » comprend aussi dans le cadre de la présente invention les sels de l'acide hyaluronique et notamment les sels alcalins comme le sel de sodium et le sel de potassium.

A l'état naturel, l'acide hyaluronique est présent dans les gels péri-cellulaires, dans la substance de base des tissus conjonctifs des organes vertébrés tels que le derme et les tissus épithéliaux et en particulier dans l'épiderme, dans le liquide synovial articulaire, dans l'humeur vitrée, dans le cordon ombilical humain et dans l'apophyse cristagalli.

Ainsi, le terme « acide hyaluronique ou l'un de ses dérivés » comprend l'ensemble des fractions ou sous-unités de l'acide hyaluronique présentant un poids moléculaire notamment compris dans la fourchette de poids moléculaire rappelée ci-dessus.

Dans le cadre de la présente invention, on préfère utiliser des fractions d'acide hyaluronique ne présentant pas d'activité inflammatoire.

A titre d'illustration des différentes fractions d'acide hyaluronique, on peut se référer au document « Hyaluronan fragments : an information-rich system », R.Stern et al, European Journal of Cell Biology 58 (2006) 699-715, qui passe en revue les activités biologiques répertoriées de l'acide hyaluronique en fonction de son poids moléculaire.

Selon un mode de réalisation privilégié de l'invention, les fractions d'acide hyaluronique adaptées à l'application visée par la présente invention présentent un poids moléculaire compris entre 50 000et 5 000 000, en particulier entre 100 000 et 5 000 000, notamment entre 400 000 et 5 000 000 Da. On parle dans ce cas d'acide hyaluronique de haut poids moléculaire.

Alternativement, les fractions d'acide hyaluronique pouvant également être adaptées à l'application visée par la présente invention présentent un poids moléculaire compris entre 50 000 et 400 000 Da. On parle dans ce cas d'acide hyaluronique de poids moléculaire intermédiaire.

Alternativement encore, les fractions d'acide hyaluronique pouvant être adaptées à l'application visée par la présente invention présentent un poids moléculaire inférieur à 50 000 Da. On parle dans ce cas d'acide hyaluronique de bas poids moléculaire.

Enfin, le terme « acide hyaluronique ou l'un de ses dérivés » comprend également les esters de l'acide hyaluronique notamment ceux dans lesquels tout ou partie des groupes carboxyliques des fonctions acide sont estérifiés avec des alcools ou des alkyles oxyéthylénés, comportant de 1 à 20 atomes de carbone, notamment avec un taux de substitution au niveau de l'acide D-glucuronique de l'acide hyaluronique variant de 0,5 à 50%.

On peut notamment citer les esters méthylique, éthylique, n-propylique, n-pentyl, benzyl et dodécyl de l'acide hyaluronique. De tels esters ont notamment été décrits dans D. Campoccia et al. « Semisynthetic resorbable materials from hyaluronan esterification », Biomaterials 19 (1998) 2101-2127.

Les poids moléculaires indiqués plus haut sont également valables pour les esters de l'acide hyaluronique.

De l'acide hyaluronique peut notamment être fourni par la société HYACTIVE sous le nom commercial CPN (PM : 10 à 150 kDa), par le société SOLIANCE sous le nom commercial CRISTALHYAL (PM : 1,1. 10⁶), par la société BIOLAND sous le nom NUTRA HA (PM : 820 000 Da), par la société BIOLAND sous le nom NUTRA AF (PM : 69 000 Da, par la société BIOLAND sous le nom OLIGO HA (PM : 6 100 Da) ou encore par la société VAM FARMACOS METICA sous le nom D FACTOR (PM : 380 Da).

Dans un mode de réalisation, l'acide hyaluronique est présent sous forme de sphères. En particulier, de telles sphères sont commercialisées par la société BASF sous le nom SPHERE d'ACIDE HYALURONIQUE. Il s'agit d'un mélange d'acide hyaluronique de différents poids moléculaires, à savoir de PM 1,5.10⁶, 400000 et 600000 Da.

L'acide hyaluronique ou l'un de ses dérivés est présent dans la composition selon la présente invention dans une teneur comprise entre 0,001 et 20 %, de préférence entre 0,01 et 10 % et plus particulièrement entre 0,01 et 5 % en poids, par rapport au poids total de la composition.

### DERIVES C-GLYCOSIDES

Un dérivé C-glycoside convenant à l'invention peut être un composé de formule générale (I) suivante : 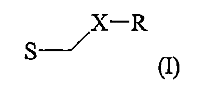 dans laquelle :
- X représente un groupement -CH(OH)-,
- S représente le D-xylose,
- la liaison, S-CH₂-X représente une liaison de nature C-anomérique, qui peut être α ou β.

Les sels acceptables pour l'usage non thérapeutique des composés décrits dans la présente invention comprennent des sels non toxiques conventionnels desdits composés tels que ceux formés à partir d'acides organiques ou inorganiques. A titre d'exemple, on peut citer les sels d'acides minéraux, tels que l'acide sulfurique, l'acide chlorhydrique, l'acide bromhydrique, l'acide iodhydrique, l'acide phosphorique, l'acide borique. On peut également citer les sels d'acides organiques, qui peuvent comporter un ou plusieurs groupes acide carboxylique, sulfonique, ou phosphonique. Il peut s'agir d'acides aliphatiques linéaires, ramifiés ou cycliques ou encore d'acides aromatiques. Ces acides peuvent comporter, en outre, un ou plusieurs hétéroatomes choisis parmi O et N, par exemple sous la forme de groupes hydroxyle. On peut notamment citer l'acide propionique, l'acide acétique, l'acide téréphtalique, l'acide citrique et l'acide tartrique.

Lorsque le composé de formule (I) comporte un groupe acide, la neutralisation du ou des groupes acides peut être effectuée par une base minérale, telle que LiOH, NaOH, KOH, Ca(OH)₂, NH₄OH, Mg(OH)₂ ou Zn(OH)₂; ou par une base organique telle qu'une alkylamine primaire, secondaire ou tertiaire, par exemple la triéthylamine ou la butylamine. Cette alkylamine primaire, secondaire ou tertiaire peut comporter un ou plusieurs atomes d'azote et/ou d'oxygène et peut donc comporter par exemple une ou plusieurs fonctions alcool; on peut notamment citer l'amino-2-méthyl-2-propanol, la triéthanolamine, la diméthylamino-2-propanol, le 2-amino-2-(hydroxyméthyl)-1,3-propanediol. On peut encore citer la lysine ou la 3-(diméthylamino)propylamine.

Les solvates acceptables pour les composés décrits dans la présente invention comprennent des solvates conventionnels tels que ceux formés lors de la dernière étape de préparation desdits composés du fait de la présence de solvants. A titre d'exemple, on peut citer les solvates dus à la présence d'eau ou d'alcools linéaires ou ramifiés comme l'éthanol ou l'isopropanol.

Les dérivés C-glycosides de formule (I), utilisés selon l'invention, sont :
1. C-β-D-xylopyranoside-2-hydroxy-propane ;
2. C-α-D-xylopyranoside-2-hydroxy-propane ;

Selon un mode de réalisation particulier, le dérivé C-glycoside peut être le C-β-D-xylopyranoside-2-hydroxy-propane sous forme d'une solution à 30 % en poids en matière active dans un mélange eau/propylène glycol (60/40 % en poids) tel que le produit fabriqué par CHIMEX sous la dénomination commerciale « MEXORYL SBB^{®} ».

Bien entendu, selon l'invention, un dérivé C-glycoside répondant à la formule (I) peut être utilisé seul ou en mélange avec d'autres dérivés C-glycosides et en toutes proportions.

Un dérivé C-glycoside convenant à l'invention peut notamment être obtenu par la méthode de synthèse décrite dans le document WO 02/051828.

La quantité de dérivé C-glycoside à mettre en oeuvre dans une composition selon l'invention dépend de l'effet cosmétique ou thérapeutique recherché, et peut donc varier dans une large mesure.

L'homme de l'art peut aisément, sur la base de ses connaissances générales, déterminer les quantités appropriées.

Une composition selon l'invention peut comprendre un dérivé C-glycoside à raison d'environ 0,0001 % à environ 25 % en poids de matière active par rapport au poids total de la composition, et en particulier d'environ 0,001 % à environ 10 % en poids de matière active, et plus particulièrement d'environ 0,05 % à environ 5 % en poids de matière active de dérivé C-glycoside par rapport au poids total de la composition.

Dans le cadre de la présente invention, le rapport pondéral entre l'acide hyaluronique ou l'un de ses dérivés et le dérivé C-glycoside, dans la composition, peut varier entre 0,001 et 1000, en particulier entre 1 et 10, et tout particulièrement entre 2 et 4.

### Galénique

L'association d'au moins un dérivé C-glycoside et d'au moins un acide hyaluronique ou de l'un de ses dérivés selon l'invention est de façon préférée formulée pour une administration par voie topique, c'est-à-dire contenant un milieu cosmétiquement ou dermatologiquement acceptable, soit un milieu compatible avec la peau, les ongles, les muqueuses, les tissus, le cuir chevelu et/ou les cheveux.

Il est clair aussi que la quantité efficace des actifs correspond à la quantité nécessaire pour obtenir le résultat désiré, et que la formulation des compositions suivant l'invention dépend de l'usage auquel ces compositions sont destinées.

En particulier, deux grandes catégories de compositions topiques suivant l'invention peuvent être distinguées, en fonction des conditions dans lesquelles elles seront appliquées sur la peau.

La première catégorie correspond aux compositions cosmétiques, c'est-à-dire destinées à être appliquées sur une peau saine afin d'en améliorer l'esthétique et notamment le confort. Une peau saine se définit par l'absence de pathologies telles que des infections, une inflammation, un érythème, ou de blessures telles qu'une brûlure ou une coupure. Cependant, dans cette définition, une peau saine ne signifie pas une peau en parfait état.

En particulier, une peau saine peut présenter des signes de sécheresse qui peuvent être d'origine exogène (la peau devient sèche, par exemple, lors d'une exposition à un air sec et très froid), ou d'origine physiologique endogène (par exemple, au moment de la chute hormonale liée à la ménopause).

La composition cosmétique est ainsi formulée pour être appliquée sur la peau et/ou ses annexes et comprend au moins un acide hyaluronique ou l'un de ses dérivés et au moins un dérivé C-glycoside, ladite composition permettant d'améliorer la fonction barrière.

Selon l'invention, l'association du dérivé C-glycoside et de l'acide hyaluronique ou de l'un de ses dérivés peut aussi être utilisé pour la préparation d'une composition thérapeutique plus précisément dermatologique, destinée à améliorer l'état d'une peau lésée, de type peau nécessitant une réparation et/ou une régénération tissulaire au niveau du derme, de l'épiderme et de la jonction dermo-épidermique.

La lésion considérée peut être d'origine quelconque, par exemple infectieuse, allergique, nerveuse ou traumatique. Une composition thérapeutique peut être appliquée directement sur l'endroit lésé ou dans son voisinage.

Ainsi, l'invention porte aussi sur l'utilisation de l'association d'au moins un dérivé C-glycoside et d'au moins un acide hyaluronique ou d'au moins l'un de ses dérivés pour la préparation d'une composition topique formulée pour un usage thérapeutique, par exemple sur une peau lésée telle qu'une peau nécessitant une réparation tissulaire au niveau du derme, de l'épiderme et de la jonction dermo-épidermique.

La composition sera alors formulée avec un véhicule pharmaceutiquement acceptable.

Selon un mode préféré de réalisation de l'invention, la composition a un pH proche de celui de la peau, compris entre 4 et 7.

Lorsqu'elle est appliquée par voie topique, la composition selon l'invention, peut être appliquée sur le visage, le cou, le cuir chevelu, les muqueuses et les ongles ou tout autre zone cutanée du corps dont les mains et les pieds.

Par « milieu physiologique acceptable », on entend désigner un milieu compatible avec la peau et/ou ses annexes ou les matières et/ou les fibres kératiniques d'êtres humains, comme par exemple, de manière non limitative, la peau, les muqueuses, les ongles, le cuir chevelu et/ou les cheveux.

Ce milieu physiologiquement acceptable comprend de l'eau, éventuellement en mélange ou non avec un ou plusieurs solvants organiques tels que des alcools en C₁-C₈, notamment l'éthanol, l'isopropanol, le tert-butanol, le n-butanol, des polyols comme la glycérine, le propylène glycol, le butylène glycol et des éthers de polyol.

Elles se présentent notamment sous forme de solutions aqueuses, hydroalcooliques ou huileuses, de dispersions du type lotion ou sérum, de gels anhydres ou huileux, d'émulsions de consistance liquide ou semi-liquide du type lait, obtenues par dispersion d'une phase grasse dans une phase aqueuse (H/E) ou inversement (E/H), d'émulsions multiples (triple : E/H/E ou H/E/H), de suspensions ou d'émulsions de consistance molle, semi-solide ou solide du type crème, gel, notamment aqueux, de micro-émulsions, de dispersions d'une phase grasse dans une phase aqueuse à l'aide de nanoparticules polymériques telles que les nanosphères et les nanocapsules, ou de vésicules lipidiques de type ionique et/ou non ionique (liposomes, niosomes, oléosomes), de nanoémulsions, ou de films fins, ou encore de micro-capsules, de micro-particules. On peut également utiliser une composition selon l'invention sous la forme d'un biphase eau et huile.

Les quantités des différents constituants des compositions utilisées selon l'invention sont celles classiquement utilisées dans les domaines considérés.

Ces compositions sont préparées selon les méthodes usuelles.

En outre, les compositions utilisées selon l'invention peuvent être plus ou moins fluides et avoir l'aspect d'une crème blanche ou colorée, d'une pommade, d'un lait, d'une lotion, d'un sérum, d'une pâte, d'une mousse. Elles peuvent être éventuellement appliquées sur la peau sous forme d'aérosol. Elles peuvent aussi se présenter sous forme solide, et par exemple sous forme de stick.

Selon un mode particulier de l'invention, la composition se présente sous forme d'un gel transparent. En particulier, ce gel transparent se caractérise par le fait qu'il est obtenu en associant les actifs avec un homopolymère d'un monomère à groupement sulfonique. Plus particulièrement, ledit gel contient de 0 à 1 % d'huile.

Les polymères comportant au moins un monomère à groupement sulfonique, utilisés dans ce type de gel transparent, sont avantageusement hydrosolubles ou hydrodispersibles ou gonflables dans l'eau. Les polymères utilisés pour ce type de gel transparent sont des homopolymères susceptibles d'être obtenus à partir d'au moins un monomère à insaturation éthylénique et à groupement sulfonique, pouvant être sous forme libre ou partiellement ou totalement neutralisée.

De façon préférentielle, les polymères utilisés pour ce type de gel transparent peuvent être neutralisés partiellement ou totalement par une base minérale (soude, potasse, ammoniaque) ou une base organique telle que la mono-, di- ou tri-éthanolamine, un aminométhylpropanediol, la N-méthyl-glucamine, les acides aminés basiques comme l'arginine et la lysine, et les mélanges de ces composés. Ils sont généralement neutralisés. On entend dans la présente invention par « neutralisés », des polymères totalement ou pratiquement totalement neutralisés, c'est-à-dire neutralisés à au moins 90 %.

Ces polymères ont généralement un poids moléculaire moyen en nombre allant de 1000 à 20 000 000 g/mole, de préférence allant de 20 000 à 5 000 000 et plus préférentiellement encore de 100 000 à 1 500 000 g/mole.

Ces polymères selon l'invention peuvent être réticulés ou non réticulés.

Les monomères à groupement sulfonique du polymère utilisés dans ces gels transparents sont choisis notamment parmi l'acide vinylsulfonique, l'acide styrènesulfonique, les acides (méth)acrylamido-(C₁-C₂₂)alkylsulfoniques, les acides N-(C₁-C₂₂)alkyl-(méth)acrylamido-(C₁-C₂₂)-alkylsulfoniques comme l'acide undécyl-acrylamido-méthane-sulfonique, ainsi que leurs formes partiellement ou totalement neutralisées, et leurs mélanges.

Selon un mode préféré de réalisation de l'invention, les monomères à groupement sulfonique sont choisis parmi les acides (méth)acrylamido(C₁-C₂₂)alkylsulfoniques tels que par exemple l'acide acrylamido-méthane-sulfonique, l'acide acrylamido-éthane-sulfonique, l'acide acrylamido-propane-sulfonique, l'acide 2-acrylamido-2-méthylpropane-sulfonique, l'acide 2-méthacrylamido-2-méthylpropane-sulfonique, l'acide 2-acrylamido-n-butane-sulfonique, l'acide 2-acrylamido-2,4,4-triméthylpentane-sulfonique, l'acide 2-méthacrylamido-dodécyl-sulfonique, l'acide 2-acrylamido-2,6-diméthyl-3-heptane-sulfonique, ainsi que leurs formes partiellement ou totalement neutralisées, et leurs mélanges.

Plus particulièrement, on utilise l'acide 2-acrylamido-2-méthylpropane-sulfonique (AMPS) ainsi que ses formes partiellement ou totalement neutralisées.

A titre d'autres polymères adaptés à ce type de gel, on peut citer notamment l'homopolymère réticulé et neutralisé d'acide 2-acrylamido 2-méthylpropane sulfonique, commercialisé par la société Clariant sous la dénomination commerciale « Hostacerin^{®} AMPS » (nom CTFA : ammonium polyacryldimethyltauramide, nom INCI : Ammonium polyacryloyle diméthyl taurate.

L'homopolymère de monomère à groupement sulfonique peut être présent dans un gel transparent en une teneur en matière active allant par exemple de 0,05 à 5 % en poids, de préférence allant de 0,1 à 5 % en poids, préférentiellement allant de 0,05 à 2 % en poids, par exemple de 0,1 à 0,4 %, notamment de 0,25 % en poids par rapport au poids total de la composition.

Quand la composition utilisée selon l'invention, quelle que soit sa nature, comporte une phase huileuse, celle-ci contient de préférence au moins une huile. Elle peut contenir en outre d'autres corps gras.

Comme huiles utilisables dans la composition de l'invention, on peut citer par exemple :
- les huiles hydrocarbonées d'origine animale, telles que le perhydrosqualène ;
- les huiles hydrocarbonées d'origine végétale, telles que les triglycérides liquides d'acides gras comportant de 4 à 10 atomes de carbone comme les triglycérides des acides heptanoïque ou octanoïque ou encore, par exemple les huiles de tournesol, de maïs, de soja, de courge, de pépins de raisin, de sésame, de noisette, d'abricot, de macadamia, d'arara, de tournesol, de ricin, d'avocat, les triglycérides des acides caprylique/caprique comme ceux vendus par la société Stearineries Dubois ou ceux vendus sous les dénominations Miglyol 810, 812 et 818 par la société Dynamit Nobel, l'huile de jojoba, l'huile de beurre de karité;
- les esters et les éthers de synthèse, notamment d'acides gras, comme les huiles de formules R¹COOR² et R¹OR² dans laquelle R¹ représente le reste d'un acide gras comportant de 8 à 29 atomes de carbone, et R² représente une chaîne hydrocarbonée, ramifiée ou non, contenant de 3 à 30 atomes de carbone, comme par exemple l'huile de Purcellin, l'isononanoate d'isononyle, le myristate d'isopropyle, le palmitate d'éthyl-2-hexyle, le stéarate d'octyl-2-dodécyle, l'érucate d'octyl-2-dodécyle, l'isostéarate d'isostéaryle ; les esters hydroxylés comme l'isostéaryl lactate, l'octylhydroxystéarate, l'hydroxystéarate d'octyldodécyle, le diisostéaryl-malate, le citrate de triisocétyle, les heptanoates, octanoates, décanoates d'alcools gras ; les esters de polyol, comme le dioctanoate de propylène glycol, le diheptanoate de néopentylglycol et le diisononanoate de diéthylèneglycol ; et les esters du pentaérythritol comme le tétraisostéarate de pentaérythrityle ;
- les hydrocarbures linéaires ou ramifiés, d'origine minérale ou synthétique, tels que les huiles de paraffine, volatiles ou non, et leurs dérivés, la vaseline, les polydécènes, le polyisobutène hydrogéné tel que l'huile de parléam ;
- les alcools gras ayant de 8 à 26 atomes de carbone, comme l'alcool cétylique, l'alcool stéarylique et leur mélange (alcool cétylstéarylique), l'octyldodécanol, le 2-butyloctanol, le 2-hexyldécanol, le 2-undécylpentadécanol, l'alcool oléique ou l'alcool linoléique ;
- les huiles fluorées partiellement hydrocarbonées et/ou siliconées comme celles décrites dans le document JP-A-2-295912 ;
- les huiles de silicone comme les polyméthylsiloxanes (PDMS) volatiles ou non à chaîne siliconée linéaire ou cyclique, liquides ou pâteux à température ambiante, notamment les cyclopolydiméthylsiloxanes (cyclométhicones) telles que la cyclohexasiloxane ; les polydiméthylsiloxanes comportant des groupements alkyle, alcoxy ou phényle, pendant ou en bout de chaîne siliconée, groupements ayant de 2 à 24 atomes de carbone ; les silicones phénylées comme les phényltriméthicones, les phényldiméthicones, les phényltriméthylsiloxydiphényl-siloxanes, les diphényldiméthicones, les diphénylméthyldiphényl trisiloxanes, les 2-phényléthyltriméthyl-siloxysilicates, et les polyméthylphénylsiloxanes ;
- leurs mélanges.

On entend par « huile hydrocarbonée » dans la liste des huiles citées ci-dessus, toute huile comportant majoritairement des atomes de carbone et d'hydrogène, et éventuellement des groupements ester, éther, fluoré, acide carboxylique et/ou alcool.

Les autres corps gras pouvant être présents dans la phase huileuse sont par exemple les acides gras comportant de 8 à 30 atomes de carbone, comme l'acide stéarique, l'acide laurique, l'acide palmitique et l'acide oléique ; les cires comme la lanoline, la cire d'abeille, la cire de Carnauba ou de Candellila, les cires de paraffine, de lignite ou les cires microcristallines, la cérésine ou l'ozokérite, les cires synthétiques comme les cires de polyéthylène, les cires de Fischer-Tropsch ; les résines de silicone telles que la trifluorométhyl-C1-4-alkyldimethicone et la trifluoropropyldimethicone ; et les élastomères de silicone comme les produits commercialisés sous les dénominations « KSG » par la société Shin-Etsu, sous les dénominations « Trefil », « BY29 » ou « EPSX » par la société Dow Corning ou sous les dénominations « Gransil » par la société Grant Industries.

Ces corps gras peuvent être choisis de manière variée par l'homme du métier afin de préparer une composition ayant les propriétés, par exemple de consistance ou de texture, souhaitées.

Selon un mode particulier de réalisation de l'invention, la composition selon l'invention est une émulsion eau-dans-huile (E/H) ou huile-dans-eau (H/E). La proportion de la phase huileuse de l'émulsion peut aller de 5 à 80 % en poids, et de préférence de 5 à 50 % en poids par rapport au poids total de la composition.

Les émulsions contiennent généralement au moins un émulsionnant choisi parmi les émulsionnants amphotères, anioniques, cationiques ou non ioniques, utilisés seuls ou en mélange, et éventuellement un co-émulsionnant. Les émulsionnants sont choisis de manière appropriée suivant l'émulsion à obtenir (E/H ou H/E). L'émulsionnant et le co-émulsionnant sont généralement présents dans la composition, en une proportion allant de 0,3 à 30 % en poids, et de préférence de 0,5 à 20 % en poids par rapport au poids total de la composition.

Pour les émulsions E/H, on peut citer par exemple comme émulsionnants les dimethicone copolyols tels que le mélange de cyclomethicone et de dimethicone copolyol, vendu sous la dénomination « DC 5225 C » par la société Dow Coming, et les alkyldimethicone copolyols tels que le Laurylmethicone copolyol vendu sous la dénomination « Dow Corning 5200 Formulation Aid » par la société Dow Corning et le Cetyl dimethicone copolyol vendu sous la dénomination Abil EM 90^{R} par la société Goldschmidt.

On peut aussi utiliser comme tensioactif d'émulsions E/H, un organopolysiloxane solide élastomère réticulé comportant au moins un groupement oxyalkyléné, tel que ceux obtenus selon le mode opératoire des exemples 3, 4 et 8 du document US-A-5,412,004 et des exemples du document US-A-5,811,487, notamment le produit de l'exemple 3 (exemple de synthèse) du brevet US-A-5,412,004. et tel que celui commercialisé sous la référence KSG 21 par la société Shin Etsu. D'autres types de KSG commercialisés par la société Shin Etsu peuvent également être utilisés, tel que le KSG-16.

Pour les émulsions H/E, on peut citer par exemple comme émulsionnants, les émulsionnants non ioniques tels que les esters d'acides gras et de glycérol oxyalkylénés (plus particulièrement polyoxyéthylénés) ; les esters d'acides gras et de sorbitan oxyalkylénés ; les esters d'acides gras oxyalkylénés (oxyéthylénés et/ou oxypropylénés) ; les éthers d'alcools gras oxyalkylénés (oxyéthylénés et/ou oxypropylénés) ; les esters de sucres comme le stéarate de sucrose ; et leurs mélanges tels que le mélange de stéarate de glycéryle et de stéarate de PEG-40.

De façon connue, la composition cosmétique ou dermatologique de l'invention peut contenir également des adjuvants habituels dans le domaine cosmétique ou dermatologique, tels que les gélifiants hydrophiles ou lipophiles, les conservateurs, les solvants, les parfums, les charges, les filtres UV, les bactéricides, les absorbeurs d'odeur, les matières colorantes, les extraits végétaux, les sels, les antioxydants, les agents basiques, les acides, les tensioactifs non ioniques, anioniques, cationiques.

Les quantités de ces différents adjuvants sont celles classiquement utilisées dans le domaine considéré, et par exemple de 0,01 à 20 % du poids total de la composition. Ces adjuvants, selon leur nature, peuvent être introduits dans la phase grasse, dans la phase aqueuse et/ou dans les vésicules lipidiques.

Comme charges qui peuvent être utilisées dans la composition de l'invention, on peut citer par exemple, outre les pigments, la poudre de silice, une silice amorphe colloïdale; le talc ; les particules de polyamide et notamment celles vendues sous la dénomination ORGASOL par la société Atochem ; les poudres de polyéthylène ; les micro-sphères à base de copolymères acryliques, telles que celles en copolymère diméthacrylate d'éthylène glycol/ methacrylate de lauryle vendues par la société Dow Corning sous la dénomination de POLYTRAP ; les poudres expansées telles que les microsphères creuses et notamment, les microsphères commercialisées sous la dénomination EXPANCEL par la société Kemanord Plast ou sous la dénomination MICROPEARL F 80 ED par la société Matsumoto ; les microbilles de résine de silicone telles que celles commercialisées sous la dénomination TOSPEARL par la société Toshiba Silicone ; et leurs mélanges. Ces charges peuvent être présentes dans des quantités allant de 0 à 20 % en poids et de préférence de 1 à 10 % en poids par rapport au poids total de la composition.

Comme gélifiants hydrophiles ou lipophiles, on peut citer notamment les carbopol, les luvigel, l'Hostacerin AMPS, le Simulgel, les gélifiants acrylamides de type Sepigel tel que le Sepigel 305^{®} de Seppic, les gommes de xanthane, de guar, de cellulose, les alginates et leurs mélanges. On peut citer aussi les hectorites.

Comme agents antioxydants, on peut citer notamment les polyphénols, l'acide tannique, l'époigallocathéchines et les extraits naturels en contenant, les anthocyanes, les extraits de romarin, les extraits de feuilles d'olivier, le thé vert, le resvératrol et ses dérivés, le Pycnogénol, l'ergothinéine, la N acétylcystéine, la biotine, les chélatants, l'idébénone, des extraits végétaux come le Pronalen Bioprotect TM de la société Provital, les antiradicalaires comme la vitamine E, le co enzyme Q10, les bioflavonoides, les SOD, le phytantriol, les lignanes, la mélatonine, les pidolates, le gluthation.

Selon un mode préféré de réalisation de l'invention, la composition utilisée selon l'invention contient au moins un filtre UV (ou filtre solaire) qui peut être un filtre chimique ou un filtre physique ou un mélange de tels filtres.

A titre d'illustration et de façon non limitative, on peut citer les familles suivantes (les noms correspondent à la nomenclature CTFA des filtres) :
les anthranilates, en particulier l'anthranilate de menthyle ; les benzophénones, en particulier la benzophénone-1, la benzophénone-3, la benzophénone-5, la benzophénone-6, la benzophénone-8, la benzophénone-9, la benzophénone-12, et préférentiellement la Benzophénone-2 (Oxybenzone), ou la Benzophénone-4 (Uvinul MS40 disponible chez B.A.S.F.); les benzylidènes-camphres, en particulier le 3-benzylidène-camphre, l'acide benzylidènecampho-sulfonque, le benzalkoniumméthosulfate de Camphre, le polyacrylamidométhylbenzylidène camphre, l'acide téréphthalylidène di-camphre sulfonique, et préférentiellement le 4-méthylbenzylidène camphre (Eusolex 6300 disponible chez Merck) ; les benzimidazoles, en particulier le benzimidazilate (Neo Heliopan AP disponible chez Haarmann et Reimer), ou l'acide phénylbenzimidazole sulfonique (Eusolex 232 disponible chez Merck) ; les benzotriazoles, en particulier le drométrizole trisiloxane, ou le méthylène bis-benzotriazolyltétraméthylbutylphénol (Tinosorb M disponible chez Ciba) ; les cinnamates, en particulier le cinoxate, le DEA méthoxycinnamate, le méthylcinnamate de diisopropyle, le glycéryl éthylhexanoate de diméthoxycinnamate, le méthoxycinnamate d'isopropyle, le cinnamate d'isoamyle, et préférentiellement l'éthocrylène (Uvinul N35 disponible chez B.A.S.F.), l'octylméthoxycinnamate (Parsol MCX disponible chez Hoffmann La Roche), ou l'octocrylène (Uvinul 539 disponible chez B.A.S.F.) ; les dibenzoylméthanes, en particulier le butyl méthoxydibenzoylméthane (Parsol 1789) ; les imidazolines, en particulier l'éthylhexyl diméthoxybenzylidène dioxoimidazoline ; les PABA, en particulier l'éthyl Dihydroxypropyl PABA, l'éthylhexyldiméthyl PABA, le glycéryl PABA, le PABA, le PEG-25 PABA, et préférentiellement la diéthylhexylbutamido-triazone (Uvasorb HEB disponible chez 3V Sigma), l'éthylhexyltriazone (Uvinul T150 disponible chez B.A.S.F.), ou l'éthyl PABA (benzocaïne) ; les salicylates, en particulier le salicyclate de dipropylèneglycol, le salicylate d'éthylhexyle, l'homosalate, ou le TEA salicylate ; les triazines, en particulier l'anisotriazine (Tinosorb S disponible chez Ciba) ; le drometrizole trisiloxane, l'oxyde de zinc, le dioxyde de titane, l'oxyde de zinc, de fer, de zirconium, de cerium enrobés ou non.

La quantité de filtres dépend de l'utilisation finale souhaitée. Elle peut aller par exemple de 1 à 20% en poids et mieux de 2 à 10% en poids par rapport au poids total de la composition.

Les compositions selon l'invention pourront être appliquées directement sur la peau ou, de façon alternative, sur des supports cosmétiques ou dermatologiques de type occlusif ou non occlusif, destinés à être appliqués de façon localisée sur la peau.

Le support peut être un support « occlusif ». A titre d'exemple, le support est constitué d'un matériau thermoplastique, choisi parmi les polyéthylènes haute et basse densité, les polypropylènes, les polychlorures de vinyle, les copolymères d'éthylène et d'acétate de vinyle, les polyesters et les polyuréthanes, ou d'un complexe de tels matériaux. Ces matériaux peuvent également être présents sous forme stratifiée avec au moins une feuille de métal telle qu'une feuille d'aluminium.

La couche support peut être de toute épaisseur appropriée qui procurera les fonctions de support et de protection souhaitées. De préférence, l'épaisseur de la couche support est comprise entre environ 20 µm et environ 1,5 mm. Avantageusement, la couche support est suffisamment flexible de manière à pouvoir épouser parfaitement le profil de la peau, et à ne pas provoquer chez l'utilisateur, une sensation d'inconfort.

De préférence toutefois, le support est « non occlusif ». Dans cette dernière hypothèse, on utilise avantageusement un support constitué d'un papier, d'un matériau thermoplastique poreux ou perforé, d'un tissé, d'un non tissé, d'un non tissé perforé.

Selon un autre mode de réalisation de l'invention, lesdites compositions selon l'invention peuvent être associées à des compositions administrées par voie orale, contenant des actifs cosmétiques additionnels à effet bénéfique sur l'aspect de la peau, tels que par exemple des actifs additionnels destinés à lutter contre les signes du vieillissement cutané ou des actifs additionnels destinés à lutter contre la peau grasse.

### Actifs additionnels

La composition selon l'invention peut en outre contenir d'autres actifs, et notamment au moins un composé choisi parmi : les agents hydratants ; les agents dépigmentants ; les agents anti-âge/anti-ride (les agents anti-glycation ; les inhibiteurs de NO-synthase ; les agents stimulant la synthèse de macromolécules dermiques ou épidermiques et/ou empêchant leur dégradation ; les agents stimulant la prolifération des fibroblastes et/ou des kératinocytes ou stimulant la différenciation des kératinocytes ; les agents myorelaxants) ; les agents ayant un effet restructurant de la fonction barrière cutanée ; les agents favorisant la maturation de l'enveloppe cornée ; les agents favorisant la microcirculation cutanée ; les agents stimulant le métabolisme énergétique cellulaire des cellules ; les agents tenseurs ; les agents antioxydants ; les agents anti-pollution et/ou anti-radicalaire, les agents desquamants, les filtres solaires, et leurs mélanges.

Par « agent hydratant », on entend :
- soit un composé agissant sur la fonction barrière, en vue de maintenir l'hydratation du stratum corneum, ou un composé occlusif. On peut citer les céramides, les composés à base sphingoïde, les lécithines, les glycosphingolipides, les phospholipides, le cholestérol et ses dérivés, les phytostérols (stigmastérol, β-sitostérol, campestérol), les acides gras essentiels, le 1-2 diacylglycérol, la 4-chromanone, les triterpènes pentacycliques tels que l'acide ursolique, la vaseline et la lanoline ;
- soit un composé augmentant directement la teneur en eau du stratum corneum, tel que le thréalose et ses dérivés, l'acide hyaluronique et ses dérivés, le glycérol, le pentanediol, le pidolate de sodium, la sérine, le xylitol, le lactate de sodium, le polyacrylate de glycérol, l'ectoïne et ses dérivés, le chitosane, les oligo- et polysaccharides, les carbonates cycliques, l'acide N-lauroyl pyrrolidone carboxylique, et la N-α-benzoyl-L-arginine ;
- soit un composé activant les glandes sébacées tel que la DHEA, ses dérivés 7-oxydés et/ou 17-alkylés, les sapogénines et la vitamine D et ses dérivés.

Les « agents dépigmentants » susceptibles d'être incorporés dans la composition selon la présente invention comprennent par exemple les composés suivants : l'acide kojique ; l'acide ellagique ; l'arbutine et ses dérivés tels que ceux décrits dans les demandes EP-895 779 et EP-524 109 ; l'hydroquinone ; les dérivés d'aminophénol tels que ceux décrits dans les demandes WO 99/10318 et WO 99/32077, et en particulier le N-cholestéryloxycarbonyl-para-aminophénol et le N-éthyloxycarbonyl-para-aminophénol ; les dérivés d'iminophénol, en particulier ceux décrits dans la demande WO 99/22707 ; l'acide L-2-oxothiazolidine-4-carboxylique ou procystéine, ainsi que ses sels et esters ; l'acide ascorbique et ses dérivés, notamment le glucoside d'ascorbyle ; et les extraits de plantes, en particulier de réglisse, de mûrier et de scutellaire, sans que cette liste soit limitative.

Par « agent anti-glycation », on entend un composé prévenant et/ou diminuant la glycation des protéines de la peau, en particulier des protéines du derme telles que le collagène.

Des exemples d'agents anti-glycation sont les extraits végétaux de la famille des Ericaceae, tels qu'un extrait de myrtille (Vaccinium angustifolium) ; l'ergothionéine et ses dérivés ; et les hydroxystilbènes et leurs dérivés, tels que le resvératrol et le 3,3', 5,5'-tétrahydroxystilbène.

Des exemples d'« inhibiteurs de NO-synthase» convenant à une utilisation dans la présente invention comprennent notamment un extrait de végétal de l'espèce *Vitis vinifera* qui est notamment commercialisé par la société Euromed sous la dénomination Leucocyanidines de raisins extra, ou encore par la société Indena sous la dénomination Leucoselect®, ou enfin par la société Hansen sous la dénomination Extrait de marc de raisin ; un extrait de végétal de l'espèce Olea europaea qui est de préférence obtenu à partir de feuilles d'olivier et est notamment commercialisé par la société VINYALS sous forme d'extrait sec, ou par la société Biologia & Technologia sous la dénomination commerciale Eurol BT ; et un extrait d'un végétal de l'espèce Gingko biloba qui est de préférence un extrait aqueux sec de ce végétal vendu par la société Beaufour sous le nom commercial Ginkgo biloba extrait standard.

Parmi les « agents stimulant les macromolécules du derme ou empêchant leur dégradation », on peut citer ceux qui agissent :
- soit sur la synthèse du collagène, tels que les extraits de *Centella asiatica* ; les asiaticosides et dérivés ; l'acide ascorbique ou vitamine C et ses dérivés ; les peptides de synthèse tels que la iamin, le biopeptide CL ou palmitoyloligopeptide commercialisé par la société SEDERMA ; les peptides extraits de végétaux, tels que l'hydrolysat de soja commercialisé par la société COLETICA sous la dénomination commerciale Phytokine® ; et les hormones végétales telles que les auxines et les lignanes.
- soit sur la synthèse d'élastine, tels que l'extrait de Saccharomyces Cerivisiae commercialisé par la société LSN sous la dénomination commerciale Cytovitin® ; et l'extrait d'algue *Macrocystis pyrifera* commercialisé par la société SECMA sous la dénomination commerciale Kelpadelie® ;
- soit sur la synthèse des glycosaminoglycanes, tels que le produit de fermentation du lait par lactobacillus vulgaris, commercialisé par la société BROOKS sous la dénomination commerciale Biomin yogourth® ; l'extrait d'algue brune *Padina pavonica* commercialisé par la société ALBAN MÜLLER sous la dénomination commerciale HSP3® ; et l'extrait de *Saccharomyces cerevisiae* disponible notamment auprès de la société SILAB sous la dénomination commerciale Firmalift® ou auprès de la société LSN sous la dénomination commerciale Cytovitin® ;
- soit sur la synthèse de la fibronectine, tels que l'extrait de zooplancton *Salina* commercialisé par la société SEPORGA sous la dénomination commerciale GP4G® ; l'extrait de levure disponible notamment auprès de la société ALBAN MÜLLER sous la dénomination commerciale Drieline® ; et le palmitoyl pentapeptide commercialisé par la société SEDERMA sous la dénomination commerciale Matrixil® ;
- soit sur l'inhibition des métalloprotéinases (MMP) telles que plus particulièrement les MMP 1, 2, 3, 9. On peut citer : les rétinoïdes et dérivés, les oligopeptides et les lipopeptides, les lipoaminoacides, l'extrait de malt commercialisé par la société COLETICA sous la dénomination commerciale Collalift® ; les extraits de myrtille ou de romarin ; le lycopène ; les isoflavones, leurs dérivés ou les extraits végétaux en contenant, en particulier les extraits de soja (commercialisé par exemple par la société ICHIMARU PHARCOS sous la dénomination commerciale Flavostérone SB®), de trèfle rouge, de lin, de kakkon ou de sauge ;
- soit sur l'inhibition des sérine protéases telles que l'élastase leucocytaire ou la cathepsine G. On peut citer : l'extrait peptidique de graines de légumineuse (Pisum sativum) commercialisé par la société LSN sous la dénomination commerciale Parelastyl^{®} ; les héparinoïdes ; et les pseudodipeptides tels que l'acide {2-[acétyl-(3-trifluorométhylphényl)-amino]-3-méthyl-butyrylamino} acétique.

Parmi les « actifs stimulant les macromolécules épidermiques », telles que la fillagrine et les kératines, on peut citer notamment l'extrait de lupin commercialisé par la société SILAB sous la dénomination commerciale Structurine^{®} ; l'extrait de bourgeons de hêtre Fagus sylvatica commercialisé par la société GATTEFOSSE sous la dénomination commerciale Gatuline^{®} ; et l'extrait de zooplancton *Salina* commercialisé par la société SEPORGA sous la dénomination commerciale GP4G^{®}.

Les « agents stimulant la prolifération des fibroblastes » utilisables dans la composition selon l'invention peuvent par exemple être choisis parmi les protéines ou polypeptides végétaux, extraits notamment du soja (par exemple un extrait de soja commercialisé par la société LSN sous la dénomination Eleseryl SH-VEG 8^{®} ou commercialisé par la société SILAB sous la dénomination commerciale Raffermine^{®}) ; et les hormones végétales telles que les giberrellines et les cytokinines.

Les « agents stimulant la prolifération des kératinocytes », utilisables dans la composition selon l'invention, comprennent notamment les rétinoïdes tels que le rétinol et ses esters, dont le palmitate de rétinyle ; le phloroglucinol ; les extraits de tourteaux de noix commercialisés par la société GATTEFOSSE ; et les extraits de Solanum tuberosum commercialisés par la société SEDERMA.

Les « agents stimulant la différenciation des kératinocytes » comprennent par exemple les minéraux tels que le calcium ; l'extrait de lupin commercialisé par la société SILAB sous la dénomination commerciale Photopréventine^{®}; le beta-sitosteryl sulfate de sodium commercialisé par la société SEPORGA sous la dénomination commerciale Phytocohésine^{®}; et l'extrait de maïs commercialisé par la société SOLABIA sous la dénomination commerciale Phytovityl^{®} ; et les lignanes tels que le sécoisolaricirésinol.

La composition selon l'invention peut comprendre des « agents dermo-décontractants », parmi lesquels on peut citer en particulier l'alvérine et ses sels, notamment le citrate d'alvérine, les sapogénines telles que la diosgénine et les extraits naturels en contenant (tels que les extraits de Wild Yam), certaines amines secondaires et tertiaires carbonylées, des sels organiques ou inorganiques de métaux, en particulier le gluconate de manganèse, l'adénosine, ainsi que l'hexapeptide argireline R commercialisé par la société LIPOTEC. On peut également citer certaines compositions parfumantes à effet dermo-décontractant.

Comme « agent ayant un effet restructurant de la barrière cutanée », on peut citer un extrait de *Thermus thermophilus* tel que le Vénucéane^{®} de Sederma, un extrait de rhizome d'igname sauvage (dioscorea villosa) tel que l'Actigen Y^{®} d'Active Organics, des extraits de plancton comme l'omega plancton^{®} de Secma, des extraits de levure comme le Relipidium^{®} de Coletica, un extrait de chataigne tel que la Recoverine^{®} de Silab, un extrait de bourgeon de cèdre tel que le Gatuline Zen^{®} de Gattefossé, la Phytosphingosine SLC de Degussa, l'Aquaxyl^{®} de Seppic, le Lipidessence^{®} de Coletica.

On peut encore citer notamment les céramides et dérivés, les composés à base de sphingoïdes, les glycosphingolipides, les phospholipides, le cholestérol et ses dérivés, les phytostérols, les acides gras essentiels, le diacylglycérol, la 4-chromanone et dérivés de chromon, la vaseline, la lanoline, les beures de karité, le cocoa butter, la lanoline...

Comme agents additionnels préférés favorisant la fonction barrière cutanée, on citera un extrait de *Thermus thermophilus*, un extrait de rhizome d'igname sauvage (dioscorea villosa), un extrait de levure, un extrait de chataigne, un extrait de bourgeon de cèdre et leurs mélanges.

A titre d' « agents favorisant la maturation de l'enveloppe cornée », on pourra utiliser dans les compositions de l'invention des agents qui interviennent sur la maturation de l'enveloppe cornée qui s'altère avec l'âge et induit une diminution de l'activité des transglutaminases. On peut citer par exemple l'urée et ses dérivés et en particulier l'Hydrovance^{®} de National Starch et les autres actifs mentionnés dans la demande L'OREAL FR2877220.

A titre d'« agents favorisant la microcirculation cutanée », on peut associer dans les compositions de l'invention des agents agissant sur la microcirculation cutanée afm d'éviter le ternissement du teint et/ou la formation des poches comme par exemple un extrait de thé noir tel que le Kombuchka de Sederma, le Pycnogénol, le gluconate de manganèse (Givobio GMn de Seppic), la Visnadine d'Indena, un extrait de lupin (Eclaline de Silab), l'Epaline 100 des Laboratoires carilène, un extrait de fleur de bigarade (Remoduline de ilab), la vitamine P et ses dérivés comme le Permethol de Sochibios et autres extraits (de ruscus, de marron d'inde, lierre, ginseng, mélilot...) et aussi la caféine, l'escine, l'hesperitine laurate, le sulfate de dextran, le nicotinate et dérivés, la lysine et dérivés (comme l'Asparlyne de Solabia)...

Comme agents additionnels préférés favorisant la microcirculation cutanée, on citera le Kombuchka, le gluconate de manganèse et la vitamine P et ses dérivés.

Par « agents stimulant le métabolisme énergétique des cellules », on peut associer également des actifs stimulant le métabolisme énérgétique qui se trouve ralentit lors du vieillissement, parmi lesquels on peut citer la biotine, un extrait de Saccharomyces cerevisaie tel que le Phosphovital^{®} de Sedenna, le Physiogenyl^{®} de Solabia, un mélange de gluconate de zinc, de cuivre et de magnésium tel que le Sepitonic M3^{®} de Seppic.

Par « agent tenseur », on entend un composé capable d'exercer une traction sur la peau, qui a pour effet d'estomper temporairement les irrégularités de la surface de la peau, telles que les rides et ridules.

A titre d'agents « anti-oxydants » on peut citer notamment le tocophérol et ses esters, en particulier l'acétate de tocophérol ; le BHT et le BHA.

On peut également citer les polyphénols, l'acide tannique, l'époigallocathéchines et les extraits naturels en contenant, les anthocyanes, les extraits de romarin, les extraits de feuilles d'olivier, le thé vert, le resvératrol et ses dérivés, le Pycnogénol, l'ergothinéine, la N acétylcystéine, la biotine, les chélatants, l'idébénone, des extraits végétaux come le Pronalen Bioprotect TM de la société Provital, les antiradicalaires comme la vitamine E, le co enzyme Q10, les bioflavonoides, les SOD, le phytantriol, les lignanes, la mélatonine, les pidolates, le gluthation.

Par l'expression « agent anti-pollution », on entend tout composé capable de piéger l'ozone, les composés aromatiques mono- ou polycycliques tels que le benzopyrène et/ou les métaux lourds tels que le cobalt, le mercure, le cadmium et/ou le nickel. Par « agent anti-radicalaire », on entend tout composé capable de piéger les radicaux libres.

Comme « agents piégeurs d'ozone » utilisables dans la composition selon l'invention, on peut citer en particulier la vitamine C et ses dérivés dont le glucoside d'ascorbyle ; les phénols et polyphénols, en particulier les tannins, l'acide ellagique et l'acide tannique ; l'épigallocatéchine et les extraits naturels en contenant ; les extraits de feuille d'olivier ; les extraits de thé, en particulier de thé vert ; les anthocyanes ; les extraits de romarin ; les acides phénols, en particulier l'acide chorogénique ; les stilbènes, en particulier le resvératrol ; les dérivés d'acides aminés soufrés, en particulier la S-carboxyméthylcystéine ; l'ergothionéine ; la N-acétylcystéine ; des chélatants comme la N,N'-bis-(3,4,5-triméthoxybenzyl)éthylènediamine ou l'un de ses sels, complexes métalliques ou esters ; des caroténoïdes tels que la crocétine ; et des matières premières diverses comme le mélange d'arginine, ribonucléate d'histidine, mannitol, adénosinetriphosphate, pyridoxine, phénylalanine, tyrosine et ARN hydrolysé commercialisé par les Laboratoires Sérobiologiques sous la dénomination commerciale CPP LS 2633-12F^{®}, la fraction hydrosoluble de maïs commercialisée par la société SOLABIA sous la dénomination commerciale Phytovityl^{®}, le mélange d'extrait de fumeterre et d'extrait de citron commercialisé sous la dénomination Unicotrozon C-49^{®} par la société Induchem, et le mélange d'extraits de ginseng, de pomme, de pêche, de blé et d'orge vendu par la société PROVITAL sous la dénomination commerciale Pronalen Bioprotect^{®}.

Comme « agents piégeurs de composés aromatiques mono- ou polycycliques » utilisables dans la composition selon l'invention, on peut citer en particulier les tannins tels que l'acide ellagique ; les dérivés indoles, en particulier l'indol-3-carbinol ; les extraits de thé en particulier de thé vert, les extraits de Jacinthe d'eau ou eichornia crassipes ; et la fraction hydrosoluble de maïs commercialisée par la société SOLABIA sous la dénomination commerciale Phytovityl^{®}.

Enfin, comme « agents piégeurs de métaux lourds » utilisables dans la composition selon l'invention, on peut citer en particulier les agents chélatants tels que l'EDTA, le sel pentasodique d'éthylènediamine tétraméthylène phosphonique, et la N,N'-bis-(3,4,5-triméthoxybenzyl)éthylènediamine ou l'un de ses sels, complexes métalliques ou esters ; l'acide phytique ; les dérivés de chitosan ; les extraits de thé, en particulier de thé vert ; les tannins tels que l'acide ellagique ; les acides aminés soufrés tels que la cystéine ; les extraits de Jacinthe d'eau (Eichornia crassipes) ; et la fraction hydrosoluble de maïs commercialisée par la société SOLABIA sous la dénomination commerciale Phytovityl^{®}.

Les « agents anti-radicalaires » utilisables dans la composition selon l'invention comprennent, outre certains agents anti-pollution mentionnés précédemment, la vitamine E et ses dérivés tels que l'acétate de tocophéryle ; les bioflavonoïdes ; le co-enzyme Q10 ou ubiquinone ; certaines enzymes comme la catalase, le superoxyde dismutase, la lactoperoxydase, le glutathion peroxydase et les quinones réductases ; le glutathion ; le benzylidène camphre ; les benzylcyclanones ; les naphtalénones substituées ; les pidolates ; le phytantriol ; le gamma-oryzanol ; les lignanes ; et la mélatonine.

Par « agent desquamant », on entend tout composé capable d'agir :
- soit directement sur la desquamation en favorisant l'exfoliation, tel que les β-hydroxyacides, en particulier l'acide salicylique et ses dérivés (dont l'acide n-octanoyl 5-salicylique) ; les α-hydroxyacides, tels que les acides glycolique, citrique, lactique, tartrique, malique ou mandélique ; l'urée ; l'acide gentisique ; les oligofucoses ; l'acide cinnamique ; l'extrait de Saphora japonica ; le resvératrol;
- soit sur les enzymes impliquées dans la desquamation ou la dégradation des cornéodesmosomes, telles que les glycosidases, la stratum corneum chymotryptic enzym

(SCCE) voire d'autres protéases (trypsine; chymotrypsine-like). On peut citer les agents chélatant des sels minéraux : l'EDTA ; l'acide N-acyl-N,N',N' éthylène diaminetriacétique ; les composés aminosulfoniques et en particulier l'acide N-(2-hydroxyéthyl)pipérazine-N'-2-éthane sulfonique (HEPES) ; les dérivés de l'acide 2-oxothiazolidine-4-carboxylique (procystéine) ; les dérivés d'acides alpha aminés de type glycine (tels que décrits dans EP-0 852 949, ainsi que le méthyl glycine diacétate de sodium commercialisé par BASF sous la dénomination commerciale TRILON M) ; le miel ; les dérivés de sucre tels que l'O-octanoyl-6-D-maltose et la N-acétyl glucosamine.

L'invention porte également sur un procédé de traitement cosmétique de la peau destiné à améliorer et/ou renforcer la fonction barrière de la peau comprenant au moins une étape consistant à appliquer sur la peau au moins une composition telle que définie précédemment.

Selon un premier mode, le procédé selon l'invention comprend au moins une étape consistant à appliquer sur la peau de personnes présentant une peau présentant au moins l'un des signes de vieillissement cutané rappelés précédemment au moins une composition telle que définie précédemment.

Selon un autre mode de réalisation, il comprend au moins une étape consistant à appliquer sur la peau de personnes présentant une peau ou une zone de peau agressée, en particulier la peau rasée du visage ou du corps, au moins une composition telle que définie précédemment.

### Ensemble cosmétique

Selon un autre aspect, l'invention concerne également un ensemble cosmétique comprenant :
i) un récipient délimitant au moins un compartiment, ledit récipient étant fermé par un élément de fermeture ; et
ii) une composition telle que décrite précédemment et disposée à l'intérieur dudit compartiment.

Le récipient peut être sous toute forme adéquate. Il peut être notamment sous forme d'un flacon, d'un tube, d'un pot, d'un étui, d'une boite, d'un sachet ou d'un boîtier.

L'élément de fermeture peut être sous forme d'un bouchon amovible, d'un couvercle, d'un opercule, d'une bande déchirable, ou d'une capsule, notamment du type comportant un corps fixé au récipient et une casquette articulée sur le corps. Il peut être également sous forme d'un élément assurant la fermeture sélective du récipient, notamment une pompe, une valve, ou un clapet.

Le récipient peut être associé à un applicateur. L'applicateur peut être sous forme d'un pinceau, tel que décrit par exemple dans le brevet FR 2 722 380. L'applicateur peut être sous forme d'un bloc de mousse ou d'élastomère, d'un feutre, ou d'une spatule. L'applicateur peut être libre (houppette ou éponge) ou solidaire d'une tige portée par l'élément de fermeture, tel que décrit par exemple dans le brevet US 5 492 426. L'applicateur peut être solidaire du récipient, tel que décrit par exemple le brevet FR 2 761 959.

Le produit peut être contenu directement dans le récipient, ou indirectement. A titre d'exemple, le produit peut être disposé sur un support imprégné, notamment sous forme d'une lingette ou d'un tampon, et disposé (à l'unité ou plusieurs) dans une boîte ou dans un sachet. Un tel support incorporant le produit est décrit par exemple dans la demande WO 01/03538.

L'élément de fermeture peut être couplé au récipient par vissage. Alternativement, le couplage entre l'élément de fermeture et le récipient se fait autrement que par vissage, notamment via un mécanisme à baïonnette, par encliquetage, serrage, soudage, collage, ou par attraction magnétique. Par « encliquetage » on entend en particulier tout système impliquant le franchissement d'un bourrelet ou d'un cordon de matière par déformation élastique d'une portion, notamment de l'élément de fermeture, puis par retour en position non contrainte élastiquement de ladite portion après le franchissement du bourrelet ou du cordon.

Le récipient peut être au moins pour partie réalisé en matériau thermoplastique. A titre d'exemples de matériaux thermoplastiques, on peut citer le polypropylène ou le polyéthylène.

Alternativement, le récipient est réalisé en matériau non thermoplastique, notamment en verre ou en métal (ou alliage).

Le récipient peut être à parois rigides ou à parois déformables, notamment sous forme d'un tube ou d'un flacon tube.

Le récipient peut comprendre des moyens destinés à provoquer ou faciliter la distribution de la composition. A titre d'exemple, le récipient peut être à parois déformables de manière à provoquer la sortie de la composition en réponse à une surpression à l'intérieur du récipient, laquelle surpression est provoquée par écrasement élastique (ou non élastique) des parois du récipient.

Le récipient peut être constitué d'un boîtier avec un fond délimitant au moins un logement contenant la composition, et un couvercle, notamment articulé sur le fond, et apte à recouvrir au moins en partie ledit fond. Un tel boîtier est décrit par exemple dans la demande WO 03/018423 ou dans le brevet FR 2 791 042.

Le récipient peut être équipé d'un essoreur disposé au voisinage de l'ouverture du récipient. Un tel essoreur permet d'essuyer l'applicateur et éventuellement, la tige dont il peut être solidaire. Un tel essoreur est décrit par exemple dans le brevet FR 2 792 618.

Le contenu des brevets ou demandes de brevets cités précédemment sont incorporés par référence dans la présente demande.

Selon un mode particulier, l'invention concerne un ensemble cosmétique comprenant :
- une composition A contenant au moins un dérivé C-glycoside- une composition B, conditionnée de manière séparée de la composition A, comprenant au moins un acide hyaluronique ou l'un de ses dérivés.

L'invention concerne enfin un procédé de traitement cosmétique comprenant au moins une étape d'application sur la peau et/ou ses annexes de la composition A et au moins une étape d'application sur la peau et/ou ses annexes de la composition B.

Selon un mode préféré, on applique en premier lieu la composition A et en second lieu la composition B.

Sans que cela les lient à aucune théorie, les inventeurs voient dans cet ordre d'application un avantage provenant du fait que le dérivé C-glycoside prépare la peau et/ou ses annexes à répondre de façon accrue à l'acide hyaluronique ou à ses dérivés.

Dans ce cas, les compositions A et B peuvent être conditionnées séparément à l'intérieur de deux compartiments, formés soit par deux récipients distincts, soit à l'intérieur d'un dispositif unitaire.

Par « dispositif unitaire » on entend un dispositif par lequel les deux compartiments sont solidaires l'un de l'autre. Un tel dispositif peut être obtenu par un procédé de moulage en une seule pièce des deux compartiments, notamment en un matériau thermoplastique. Il peut également résulter de toute forme d'assemblage, notamment par collage, soudage, ou autre encliquetage.

Selon un premier mode de réalisation, les deux récipients sont indépendants l'un de l'autre. De tels récipients peuvent se présenter sous diverses formes. Il peut s'agir notamment de tubes, de flacons ou de bidons.

L'un et/ou l'autre des récipients peuvent être surmontés d'une pompe à actionnement manuel surmontée d'un bouton poussoir pour l'actionnement de la pompe et la distribution de la composition *via* au moins un orifice de distribution.

Alternativement, l'un et/ou l'autre des récipients sont pressurisés, notamment au moyen d'un agent propulseur, en particulier un gaz propulseur. Dans ce cas, le (ou les) récipient(s) est (sont) équipé(s) d'une valve surmontée par un bouton poussoir équipée d'une buse ou de tout autre moyen de diffusion pour la distribution du produit.

Le propulseur peut être en mélange avec la composition à distribuer ou séparé, notamment *via* un piston apte à coulisser à l'intérieur du récipient, ou *via* les parois souples d'une poche à l'intérieur de laquelle est disposée la composition.

Les récipients peuvent être constitués de matériaux divers : plastique, verre, ou métal.

Alternativement encore, les deux compartiments sont formés de deux compartiments concentriques formés à l'intérieur d'un tube, et sont surmontés d'une pompe sans reprise d'air équipée d'un bouton poussoir à un ou deux orifices de distribution. A l'intérieur du tube est prévu un piston qui remonte en direction de la pompe au fur et à mesure que les compositions sont prélevées à l'intérieur des récipients. De tels modes de distribution sont utilisés notamment pour la distribution de pâtes dentifrices.

Les exemples qui figurent ci-après illustrent la présente invention.

### EXEMPLES

Le dérivé C-glycoside utilisé est le C-β-D-xylopyranoside-2-hydroxy-propane commercialisé sous la dénomination MEXORYL^{®} de CHIMEX. Il se présente sous la forme d'une solution à 30 % en poids en matière active dans un mélange eau/1,2propanediol 60/40. L'acide hyaluronique utilisé est le Cristalhyal^{®} AL commercialisé par la société Solliance (PM : 1,1 10⁶ Da)

### Exemple 1 : Test réalisé : dosage de l'activité transglutaminase kératinocytaire (TGk)

L'effet *in vitro* de 2 actifs a été étudié (le C-β-D-xylopyranoside-2-hydroxy-propane à 30 % en poids en matière active dans un mélange eau/1,2 propanediol 60/40 et l'acide hyaluronique), sur la différenciation kératinocytaire. Pour cela, des kératinocytes épidermiques humains normaux (NHEK), préalablement traités à la mitomycine pour empêcher leur prolifération, dans un milieu carencé composé à 40 % de milieu SFM appauvri et à 60 % de milieu tampon Earl Buffer Salt Sodium (EBSS) ont été mis en culture. Le métabolisme de ces cellules ainsi ralenti, leur capacité à se différencier est alors quantifiée en dosant à l'aide d'un test radiochimique standard l'activité de la Transglutaminase Kératinocytaire (TGk) des NHEK préalablement traités par les actifs.

Le Chlorure de Calcium (CaCl₂), molécule connue pour stimuler la différenciation des kératinocytes, a été utilisé en tant que molécule de référence pour cet essai.

### Mode opératoire

Des kératinocytes humains normaux ont été cultivés à 37 °C et 5 % CO₂ pendant 24 h en milieu SFM complet. Les divisions cellulaires ont ensuite été bloquées par traitement à la mitomycine C (10µg/ml) pendant 2 heures à 37 °C et 5 % de CO₂. Les cellules ont ensuite été incubées pendant 96 heures dans du milieu carencé ou non (contrôle) contenant ou non diverses concentrations des produits à l'essai ou la référence. En parallèle, le 1,2-propanediol, solvant du C-β-D-xylopyranoside-2-hydroxy-propane, a été testé. Les traitements ont été réalisés en triplicata (n=3).

A la fin de l'incubation, l'enzyme TGk membranaire a été extraite en présence de Triton X-100, puis l'activité TGk a été déterminée en mesurant l'addition covalente de putrescine tritiée à 2 µCi/ml final à la caséine (protéine acceptrice, 2 mg/ml final).

La caséine a été précipitée par addition d'acide trichloroacétique (TCA) et les précipités ont été récupérés sur filtres et collecteur puis le comptage des filtres secs en scintillation liquide a été réalisé.

### Résultats

### Activité TGk - Milieu avec mitomycine

| **TRAITEMENT** | **CONCENTRATION** | **% activité par rapport au témoin** | **Variation significative par rapport au témoin** |
|---|---|---|---|
| Témoin milieu carencé (40 % SFM [+ Ep + EGF] + 60 % EBSS [+Ca + Mg]) | - | 100 | - |
| Contrôle milieu normal (100 % SFM [+ Ep + EGF]) | - | 209 | **+ 109 %** |
| CaCl₂ | 1,5 mM | 557 | **+ 457 %** |
| Acide hyaluronique⁽¹⁾ | 0,02 % | 88 | NS |
| C-β-D-xylopyranoside-2-hydroxy-propane⁽²⁾ | 0,06 % | 128 | NS |
| 1,2-propanediol | 0,16 % | 87 | NS |
| Acide hyaluronique ⁽¹⁾ et C-β-D-xylopyranoside-2-hydroxy-propane⁽²⁾ | 0,02 % et 0,06 % | 222 | **+122 %** |

| | | | |
|---|---|---|---|
| (NS = non significatif) (1) Cristalhyal AL commercialisé par la société Solliance (PM : 1,1.10⁶ Da) (2) Mexoryl SBB® fabriqué par CHIMEX | | | |

Dans cet essai, le chlorure de calcium à 1,5 mM, utilisé comme molécule de référence, a augmenté nettement l'activité de la TGk des kératinocytes. Ce résultat était en accord avec les effets attendus et a permis de valider l'essai.

Le C-β-D-xylopyranoside-2-hydroxy-propane testé à 0,06% n'a pas d'effet significatif sur l'activité de la TGk.

Son solvant, le 1,2-propanediol, testé à 0,8 % n'a pas non plus modifié l'activité de cette enzyme.

L'acide hyaluronique testé seul à 0,02% est également sans effet sur l'activité de la Transglutaminase kératinocytaire.

L'association du C-β-D-xylopyranoside-2-hydroxy-propane et de l'acide hyaluronique, testé respectivement à 0,06 % et 0,02 % a présenté un effet synergique, dans la mesure où les produits testés seuls et à cette concentration ne modifient pas l'activité TGk alors que l'association a significativement stimulé l'activité. Les mêmes effets ont été observés lorsque l'activité était rapportée à la quantité de protéines.

### Conclusion

Le mélange C-β-D-xylopyranoside-2-hydroxy-propane/acide hyaluronique augmente l'activité de la Transglutaminase kératinocytaire dans les kératinocytes épidermiques humaines normaux cultivés en milieu carencé.

Les effets du C-β-D-xylopyranoside-2-hydroxy-propane sont potentialisés par l'acide hyaluronique et sont voisins de ceux observés si les cellules étaient cultivés en milieu normal.

Les résultats obtenus pour cette association d'actifs traduisent une augmentation de la différenciation épidermique.

### Exemple 2 : exemples de formulations

Les teneurs sont indiquées en pourcentage massique.

Les compositions A et B sont des émulsions H/E gélifiées par un carbomère et ou l'ammonium polyacryloyldiméthyl taurate et préparées selon les techniques usuelles. La composition B est très fortement siliconée. La composition C est une émulsion E/H fortement siliconée et préparée selon les techniques usuelles. La composition D est un gel transparent fortement concentré en acide hyaluronique et gélifié par l'ammonium polyacryloyldiméthyl taurate.

| | **A** | **B** | **C** | **D** |
|---|---|---|---|---|
| Sulfate de magnésium | | | 0,70 | |
| Hydroxyde de sodium | 0,08 | | | |
| Triéthanolamine | | 1,23 | 0,01 | 0,02 |
| Acide sulfonique d'hydroxyéthylpipérazine éthane | 1,00 | | | 1,00 |
| C-β-D-xylopyranoside-2-hydroxy-propane ⁽¹⁾ | 1,00 | 1,00 | 1,00 | 1,00 |
| Extraits d'algues | 1,00 | 0,20 | 0,20 | 0,20 |
| Distéardimonium hectorite | | | 0,20 | |
| Silice | | 2,00 | 3,00 | |
| Copolymère acrylate | | | 0,30 | |
| Tristéarine (et) Stéarate glycol acétylé | | | 1,00 | |
| Triglycéride caprylique/caprique | | 3,00 | | |
| Phénoxyéthanol | 0,60 | | 0,70 | |
| Méthylparaben | 0,20 | | | |
| Chlorphenesine | 0,30 | | | |
| Ethylhexyl méthoxycinnamate | 1,00 | | 1,00 | |
| Acide sulfonique de phénylbenzimidazole | | 1,80 | | |
| Ethylhéxyl méthoxycinnamate | | 7,50 | | |
| Dioxyde de titane (et) mica | 1,00 | 1,00 | 1,00 | |
| Hydroxyéthylcellulose | | | | 0,12 |
| Sodium hyaluronate⁽²⁾ | 0,02 | 0,02 | 0,02 | 0,50 |
| Carbomère | 0,15 | | | |
| Ammonium polyacryloyldiméthyl taurate | 1,00 | 1,60 | | 0,25 |
| Polyméthyle méthacrylate | | 2,00 | | |
| HDI/Triméthylol hexyllactone crosspolymère | | 1,00 | | |
| Cyclohéxasiloxane | | 3,00 | 22,00 | |
| Cétyl PEG/PPG-10/1 diméthicone | | | 3,00 | |
| Diméthicone (et) diméthicone /vinyl diméthicone crosspolymère | 10,00 | | | |
| Bis-PEG-18 méthyl ether diméthyl silane | | | | 3,00 |
| Stéaryl diméthicone | 2,00 | | | |
| Cyclopentasiloxane (et) diméthiconol | | 2,00 | | |
| Diméthicone (et) diméthiconol | | 3,00 | | |
| Cyclopentasiloxane (et) diméthicone crosspolymère | 25,00 | | | |
| Divinyldiméthicone/diméthicone crosspolymère (et) C12-13 pareth-23 (et) C12-13 pareth-3 | | 0,50 | | |
| Diméthicone (et) ceteth-10 (et) Laureth-4 | 3,50 | | | |
| Alcool dénaturé | | 5,00 | 5,00 | 7,00 |
| Eau | 38,15 | 55,15 | 45,87 | 79,41 |
| Glycérine | 7,00 | 7,00 | 10,00 | 7,00 |
| Propylène glycol | 6,00 | | 4,00 | |
| Caprylyl glycol | | | | 0,50 |
| Polysorbate 80 | 1,00 | | | |
| Méthyl glucose sesquistéarate PEG-20 | | 2,00 | | |
| Polyglycéryl-4 isostéarate | | | 1,00 | |

| | | | | |
|---|---|---|---|---|
| (1)MEXORYL SBB^{®} fabriqué par CHIMEX (2)Cristalhyal AL^{®} commercialisé par Solliance | | | | |

## Revendications

1. Composition cosmétique et/ou dermatologique topique comprenant, dans un milieu physiologiquement acceptable, au moins un acide hyaluroniqué ou l'un de ses sels alcalins, ou un ester de l'acide hyaluronique, choisi parmi les esters méthylique, éthylique, N-propylique, N-penthyl, benzyl et dodécyl de l'acide hyaluronique et au moins un dérivé C-glycoside, ledit dérivé C-glycoside répondant à la formule générale (I) suivante : 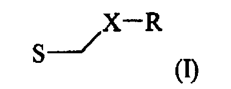 dans laquelle :
- R désigne un radical linéaire en C₁-C₄,
- X représente un groupement -CH(OH)-, et
- S représente le D-xylose,
- la liaison S-CH₂-X représente une liaison de nature C-anomérique, qui peut être α ou β,
ainsi que leurs sels cosmétiquement acceptables, et leurs solvates tels que les hydrates.

2. Composition selon la revendication précédente, dans laquelle R désigne un radical linéaire en C₁-C₃, notamment méthyle.

3. Composition selon l'une quelconque des revendications précédentes, dans laquelle le dérivé C-glycoside est choisi parmi le C-β-D-xylopyranoside-2-hydroxy-propane et le C-α-D-xylopyranoside-2-hydroxy-propane, et est plus particulièrement le C-β-D-xylopyranoside-2-hydroxy-propane.

4. Composition selon l'une quelconque des revendications précédentes, **caractérisée en ce que** le dérivé C-glycoside est présent dans une teneur allant de 0,0001 % à 25 % en poids, et en particulier de 0,001 % à 10 % en poids, et plus particulièrement de 0,05 % à 5 % en poids de matière active de dérivé C-glycoside par rapport au poids total de la composition.

5. Composition selon l'une quelconque des revendications précédentes dans laquelle l'acide hyaluronique ou l'un de ses sels alcalins, ou un ester de l'acide hyaluronique, choisi parmi les esters méthylique, éthylique, N-propylique, N-penthyl, benzyl et dodécyl de l'acide hyaluronique présente un poids moléculaire compris entre 50 000 et 5 000 000, en particulier entre 100 000 et 5 000 000, notamment entre 400 000 et 5 000 000 Da.

6. Composition selon la revendication 1 ou 2, dans laquelle l'acide hyaluronique ou l'un de ses sels alcalins, ou un ester de l'acide hyaluronique, choisi parmi les esters méthylique, éthylique, N-propylique, N-penthyl, benzyl et dodécyl de l'acide hyaluronique est présent dans une teneur comprise entre 0,001 et 20 %, en particulier entre 0,01 et 10 %, et plus particulièrement entre 0,01 et 5 % en poids de matière active d'acide hyaluronique ou de l'un de ses sels alcalins, ou d'un ester de l'acide hyaluronique, choisi parmi les esters méthylique, éthylique, N-propylique, N-penthyl, benzyl et dodécyl de l'acide hyaluronique, par rapport au poids total de la composition.

7. Composition selon l'une quelconque des revendications précédentes, dans laquelle le rapport pondéral entre l'acide hyaluronique ou l'un de ses sels alcalins, ou un ester de l'acide hyaluronique, choisi parmi les esters méthylique, éthylique, N-propylique, N-penthyl, benzyl et dodécyl de l'acide hyaluronique et le dérivé C-glycoside varie entre 0,001 et 1 000, en particulier entre 1 et 10 et tout particulièrement entre 2 et 4.

8. Ensemble cosmétique comprenant :
- une composition A, comprenant au moins un dérivé C-glycoside, tel que defini en revendication 1
- une composition B, comprenant au moins un dérivé d'acide hyaluronique ou l'un de ses sels alcalins, ou un ester de l'acide hyaluronique, choisi parmi les esters méthylique, éthylique, N-propylique, N-penthyl, benzyl et dodécyl de l'acide hyaluronique.

9. Utilisation non-therapeutique cosmétique d'une composition telle que définie selon l'une quelconque des revendications 1 à 7 ou d'ensemble cosmétique selon la revendication 8 pour améliorer et/ou renforcer la fonction barrière de la peau.

10. Utilisation non-therapeutique cosmétique d'une composition telle que définie selon l'une quelconque des revendications 1 à 7 ou d'ensemble cosmétique selon la revendication 8 pour améliorer et/ou renforcer la protection de la peau vis-à-vis d'agressions extérieures.

11. Utilisation non-therapeutique cosmétique d'une composition telle que définie selon l'une quelconque des revendications 1 à 7 ou d'ensemble cosmétique selon la revendication 8 pour améliorer l'hydratation de la peau et/ou de ses annexes.

12. Utilisation non-therapeutique cosmétique d'une composition telle que définie selon l'une quelconque des revendications 1 à 7 ou d'ensemble cosmétique selon la revendication 8 pour prévenir et/ou traiter la rugosité ou le micro-relief de la peau et/ou de ses annexes et/ou pour améliorer l'éclat du teint et/ou pour améliorer la souplesse de la peau et/ou de ses annexes.

13. Utilisation non-therapeutique cosmétique d'une composition telle que définie selon l'une quelconque des revendications 1 à 7 ou d'ensemble cosmétique selon la revendication 8 pour estomper les signes cutanés du vieillissement.

14. Utilisation cosmétique selon la revendication précédente, **caractérisée en ce que** les signes cutanés du vieillissement sont choisis parmi l'altération des propriétés viscoélastiques ou biomécaniques de la peau, l'altération de la cohésion des tissus, l'amincissement de la peau, l'apparition de rides et/ou de ridules, l'apparition d'un brunissement et/ou d'un jaunissement de la peau, et l'apparition de tâches de vieillesse ou sénescence ou lentigo.

15. Procédé de traitement cosmétique non-therapeutique destiné à améliorer et/ou renforcer la fonction barrière et/ou l'hydratation et/ou la résistance aux agressions extérieures de la peau et/ou de ses annexes et/ou pour lutter contre les signes cutanés du vieillissement et **caractérisé en ce qu'**on applique une quantité efficace d'au moins un dérivé C-glycoside tel que défini selon l'une quelconque des revendications 1 à 3 et d'au moins un acide hyaluronique ou l'un de ses sels alcalins, ou un ester de l'acide hyaluronique, choisi parmi les esters méthylique, éthylique, N-propylique, N-penthyl, benzyl et dodécyl de l'acide hyaluronique sur la peau et/ou ses annexes.

16. Procédé de traitement cosmétique comprenant au moins une étape d'application sur la peau et/ou ses annexes de la composition A telle que définie en revendication 8, et au moins une étape d'application sur la peau et/ou ses annexes de la composition B telle que définie en revendication 8, préférentiellement dans cet ordre.

17. Composition comprenant au moins un acide hyaluronique ou l'un de ses sels alcalins, ou un ester de l'acide hyaluronique, choisi parmi les esters méthylique, éthylique, N-propylique, N-penthyl, benzyl et dodécyl de l'acide hyaluronique et au moins un dérivé C-glycoside tel que défini selon l'une quelconque des revendications 1 à 3 pour être utilisée dans une méthode pour l'amélioration et/ou le renforcement de la fonction barrière d'une peau lésée, en particulier d'une peau nécessitant une réparation et/ou une régénération tissulaire.

## Claims

1. A topical cosmetic and/or dermatological composition comprising, in a physiologically acceptable medium, at least one hyaluronic acid or one of its alkaline salt thereof and at least one C-glycoside derivative,
said C-glycoside derivative corresponding to general formula (I) below: 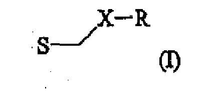 in which:
- R represents a C₁-C₄ non-substituted linear alkyl radical,
- X represents a -CH(OH)- group, and
- S represents D-xylose,
- the S-CH₂-X bond represents a bond of C-anomeric nature which may be α or β,
and also the cosmetically acceptable salts thereof, and the solvates thereof, such as hydrates.

2. The composition as claimed in claim 1, in which R represents a C₁-C₃ non-substituted linear alkyl radical, notably a methyl group.

3. The composition as claimed in anyone of the preceding claims, in which the C-glycoside derivative is chosen from C-β-D-xylopyranoside-2-hydroxypropane and C-α-D-xylopyranoside-2-hydroxypropane, and is more particularly C-β-D-xylopyranoside-2-hydroxypropane.

4. The composition as claimed in anyone of the preceding claims, **characterized in that** the C-glycoside derivative is present at a content ranging from 0.0001% to 25% by weight, in particular from 0.001% to 10% by weight, and more particularly from 0.05% to 5% by weight of active material of C-glycoside derivative, relative to the total weight of the composition.

5. The composition as claimed inanyone of the preceding claims, in which the hyaluronic acid or one of its alcaline salt thereof, or a hyaluronic acide ester, chosen from methylic, ethylic, N-propylic, N-penthyl, benzyl and dodecyl hyaluronic acid esters, has a molecular weight of between 50 000 and 5 000 000 Da, in particular of between 100 000 and 5 000 000 Da, notably of between 400 000 and 5 000 000 Da.

6. The composition as claimed in claims 1 or 2, in which the hyaluronic acid or one of its alcaline salt thereof or a hyaluronic acide ester, chosen from methylic, ethylic, N-propylic, N-penthyl, benzyl and dodecyl hyaluronic acid esters, is present at a content of between 0.001% and 20% by weight, in particular of between 0.01% and 10% by weight, and more particularly of between 0.01% and 5% by weight of active material of hyaluronic acid or one of its alcaline salt thereof, or a hyaluronic acide ester, chosen from methylic, ethylic, N-propylic, N-penthyl, benzyl and dodecyl hyaluronic acid esters, relative to the total weight of the composition.

7. The composition as claimed in anyone of the preceding claims, in which the ratio by weight of the hyaluronic acid or one of its alcaline salt thereof or a hyaluronic acide ester, chosen from methylic, ethylic, N-propylic, N-penthyl, benzyl and dodecyl hyaluronic acid esters, to the C-glycoside derivative ranges between 0.001 and 1000, in particular between 1 and 10 and more particularly between 2 and 4.

8. A cosmetic assembly comprising:
- a composition A comprising at least one C-glycoside derivative as defined in claim 1,
- a composition B comprising at least one hyaluronic acid derivative or one of its alcaline salt thereof, or a hyaluronic acide ester, chosen from methylic, ethylic, N-propylic, N-penthyl, benzyl and dodecyl hyaluronic acid esters.

9. Non-therapeutic cosmetic method for using a composition as defined in anyone of claims 1 to 7 or of a cosmetic assembly as claimed in clam 8, for improving and/or reinforcing the barrier function of the skin.

10. Non-therapeutic cosmetic method for using a composition as defined in anyone of claims 1 to 7 or of a cosmetic assembly as claimed in claim 8, for improving and/or reinforcing the protection of the skin against outside attacks.

11. Non-therapeutic cosmetic method for using a composition as defined in anyone of claims 1 to 7 or of a cosmetic assembly as claimed in claim 8, for improving the hydration of the skin and/or its appendages.

12. Non-therapeutic cosmetic method for using a composition as defined in anyone of claims 1 to 7 or of a cosmetic assembly as claimed in claim 8, for preventing and/or treating the roughness or the microrelief of the skin and/or its appendages and/or for improving the radiance of the complexion and/or for improving the suppleness of the skin and/or its appendages.

13. Non-therapeutic cosmetic method for using a cosmetic composition as defined in anyone of claims 1 to 7 or of a cosmetic assembly as claimed in claim 8, for smoothing out the signs of skin aging.

14. Method as claimed in the claim 13, **characterized in that** the signs of skin aging are chosen from impairment of the viscoelastic or biomechanical properties of the skin, impairment of tissue cohesion, thinning of the skin, the appearance of wrinkles and/or fine lines, the appearance of browning and/or of yellowing of the skin and the appearance of age or senescence or lentigo spots.

15. A non-therapeutic cosmetic treatment process for improving and/or reinforcing the barrier function and/or the hydration and/or the resistance to outside attacks of the skin and/or its appendages, and/or for combating the signs of skin aging, and **characterized in that** an effective amount of at least one C-glycoside derivative as defined in anyone of claims 1 to 3 and of at least one hyaluronic acid or a derivative thereof, or a hyaluronic acide ester, chosen from methylic, ethylic, N-propylic, N-penthyl, benzyl and dodecyl hyaluronic acid esters, is applied to the skin and/or its appendages.

16. A cosmetic treatment process comprising at least one step of applying composition A as defined in claim 8 to the skin and/or its appendages and at least one step of applying composition B as defined in claim 8 to the skin and/or its appendages, preferably in this order.

17. A composition comprising at least one hyaluronic acid or one of its alcaline salt thereof, or a hyaluronic acide ester, chosen from methylic, ethylic, N-propylic, N-penthyl, benzyl and dodecyl hyaluronic acid esters, and at least one C-glycoside derivative as defined in anyone of claims 1 to 3 to be used in a method for improving and/or reinforcing the barrier function of a damaged skin, in particular of a skin requiring tissue repair and/or regeneration.

## Patentansprüche

1. Kosmetische und/oder dermatologische Zusammensetzung, umfassend, in einem physiologisch annehmbaren Medium, mindestens eine Hyaluronsäure oder eines ihrer alkalischen Salze oder einen Ester der Hyaluronsäure, der aus den Methyl-, Ethyl-, N-Propyl-, N-Penthyl-, Benzyl- und Dodecylestern der Hyaluronsäure ausgewählt ist, und mindestens ein C-Glycosid-Derivat, wobei das C-Glycosid-Derivat der folgenden allgemeinen Formel (I) entspricht: 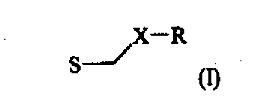 in der:
- R einen unsubstitutiert linearen C₁-C₄-Alkyl-Rest bezeichnet,
- X eine Gruppe -CH(OH)- darstellt und
- S die D-Xylose darstellt,
- Die Bindung S-CH₂-X eine Bindung C-anomerischer Natur darstellt, die α oder β sein kann,
sowie ihre kosmetisch annehmbaren Salze und ihre Solvate wie die Hydrate.

2. Zusammensetzung nach dem vorhergehenden Anspruch, bei der R einen unsubstitutiert linearen C₁-C₃-Alkyl-Rest, insbesondere Methyl, bezeichnet.

3. Zusammensetzung nach einem der vorhergehenden Ansprüche, bei der das C-Glycosid-Derivat aus C-β-D-Xylopyranosid-2-hydroxypropan und C-α-D-Xylopyranosid-2-hydroxypropan ausgewählt ist und insbesondere C-β-D-Xylopyranosid-2-hydroxypropan ist.

4. Zusammensetzung nach einem der vorhergehenden Ansprüche, **dadurch gekennzeichnet, dass** das C-Glycosid-Derivat in einem Gehalt vorliegt, der von 0,0001 bis 25 Gew.-% und besonders von 0,001 bis 10 Gew.-% und insbesondere von 0,05 bis 5 Gew.-% aktives C-Glycosidderivat-Material, bezogen auf das Gesamtgewicht der Zusammensetzung, geht.

5. Zusammensetzung nach einem der vorhergehenden Ansprüche, bei der die Hyaluronsäure oder eines ihrer alkalischen Salze oder ein Ester der Hyaluronsäure, der aus den Methyl-, Ethyl-, N-Propyl-, N-Penthyl-, Benzyl- und Dodecylestern der Hyaluronsäure ausgewählt ist, ein Molekulargewicht zwischen 50 00 und 5 000 000, besonders zwischen 100 000 und 5 000 000 und insbesondere zwischen 400 000 und 5 000 000 Da aufweist.

6. Zusammensetzung nach Anspruch 1 oder 2, bei der die Hyaluronsäure oder eines ihrer alkalischen Salze oder ein Ester der Hyaluronsäure, der aus den Methyl-, Ethyl-, N-Propyl-, N-Penthyl-, Benzyl- und Dodecylestern der Hyaluronsäure ausgewählt ist, in einem Gehalt zwischen 0,001 und 20 Gew.-%, besonders zwischen 0,01 und 10 Gew.-% und insbesondere zwischen 0,01 und 5 Gew.-% aktives Material von Hyaluronsäure oder eines ihrer alkalischen Salze oder eines Esters der Hyaluronsäure, der aus den Methyl-, Ethyl-, N-Propyl-, N-Penthyl-, Benzyl- und Dodecylestern der Hyaluronsäure ausgewählt ist, bezogen auf das Gesamtgewicht der Zusammensetzung, vorliegt.

7. Zusammensetzung nach einem der vorhergehenden Ansprüche, bei der das Gewichtsverhältnis zwischen der Hyaluronsäure oder einem ihrer alkalischen Salze oder einem Ester der Hyaluronsäure, der aus den Methyl-, Ethyl-, N-Propyl-, N-Penthyl-, Benzyl- und Dodecylestern der Hyaluronsäure ausgewählt ist, und dem C-Glycosid-Derivat zwischen 0,001 und 1 000, insbesondere zwischen 1 und 10 und ganz besonders zwischen 2 und 4 variiert.

8. Kosmetische Einheit, umfassend:
- eine Zusammensetzung A, die mindestens ein C-Glycosid-Derivat umfasst, wie es in Anspruch 1 definiert ist,
- eine Zusammensetzung B, die mindestens ein Hyaluronsäure-Derivat oder eines ihrer alkalischen Salze oder einen Ester der Hyaluronsäure umfasst, der aus den Methyl-, Ethyl-, N-Propyl-, N-Penthyl-, Benzyl- und Dodecylestern der Hyaluronsäure ausgewählt ist.

9. Nicht-therapeutische kosmetische Verwendung einer Zusammensetzung, wie sie nach einem der Ansprüche 1 bis 7 definiert ist, oder einer kosmetischen Einheit nach Anspruch 8 zur Verbesserung und/oder Verstärkung der Barrierefunktion der Haut.

10. Nicht-therapeutische kosmetische Verwendung einer Zusammensetzung, wie sie nach einem der Ansprüche 1 bis 7 definiert ist, oder einer kosmetischen Einheit nach Anspruch 8 zur Verbesserung und/oder Verstärkung des Schutzes der Haut gegen äußere Angriffe.

11. Nicht-therapeutische kosmetische Verwendung einer Zusammensetzung, wie sie nach einem der Ansprüche 1 bis 7 definiert ist, oder einer kosmetischen Einheit nach Anspruch 8 zur Verbesserung der Hydratisierung der Haut und/oder ihrer Anhänge.

12. Nicht-therapeutische kosmetische Verwendung einer Zusammensetzung, wie sie nach einem der Ansprüche 1 bis 7 definiert ist, oder einer kosmetischen Einheit nach Anspruch 8 für die Vorbeugung und/oder Behandlung der Rauheit oder des Mikroreliefs der Haut und/oder ihrer Anhänge und/oder zur Verbesserung der Leuchtkraft des Teints und/oder zur Verbesserung der Geschmeidigkeit der Haut und/oder ihrer Anhänge.

13. Nicht-therapeutische kosmetische Verwendung einer Zusammensetzung, wie sie nach einem der Ansprüche 1 bis 7 definiert ist, oder einer kosmetischen Einheit nach Anspruch 8 zum Mildern der kutanen Anzeichen des Alterns.

14. Kosmetische Verwendung nach dem vorhergehenden Anspruch, **dadurch gekennzeichnet, dass** die kutanen Anzeichen des Alterns ausgewählt sind aus der Beeinträchtigung der viskoelastischen oder biomechanischen Eigenschaften der Haut, der Beeinträchtigung der Kohäsion der Gewebe, der Verdünnung der Haut, dem Auftreten von Falten und/oder Runzeln, dem Auftreten eines Braun- und/oder Gelbwerdens der Haut und dem Auftreten von Alters- oder Seneszenzflecken oder Lentigo.

15. Nicht-therapeutisches kosmetisches Behandlungsverfahren, das dazu bestimmt ist, die Barrierefunktion und/oder die Hydratisierung und/oder den Widerstand der Haut und/oder ihrer Anhänge gegen äußere Angriffe zu verbessern und/oder zu verstärken und/oder die kutanen Anzeichen des Altems zu bekämpfen, **dadurch gekennzeichnet, dass** man eine wirksame Menge mindestens eines C-Glycosid-Derivats, wie es nach einem der Ansprüche 1 bis 3 definiert ist, und mindestens einer Hyaluronsäure oder eines ihrer alkalischen Salze oder eines Esters der Hyaluronsäure, der aus den Methyl-, Ethyl-, N-Propyl-, N-Penthyl-, Benzyl- und Dodecylestern der Hyaluronsäure ausgewählt ist, auf die Haut und/oder ihre Anhänge aufträgt.

16. Kosmetisches Behandlungsverfahren, umfassend mindestens einen Schritt des Auftragens der Zusammensetzung A, wie sie in Anspruch 8 definiert ist, auf die Haut und/oder ihre Anhänge und mindestens einen Schritt des Auftragens der Zusammensetzung B, wie sie in Anspruch 8 definiert ist, auf die Haut und/oder ihre Anhänge vorzugsweise in dieser Reihenfolge.

17. Zusammensetzung, umfassend mindestens eine Hyaluronsäure oder eines ihrer alkalischen Salze oder einen Ester der Hyaluronsäure, der aus den Methyl-, Ethyl-, N-Propyl-, N-Penthyl-, Benzyl- und Dodecylestern der Hyaluronsäure ausgewählt ist, und mindestens ein C-Glycosid-Derivat, wie es nach einem der Ansprüche 1 bis 3 definiert ist, zur Verwendung in einem Verfahren zur Verbesserung und/oder Verstärkung der Barrierefunktion einer verletzten Haut, insbesondere einer Haut, die eine Gewebereparatur und/oder -regeneration erfordert.
